# EUROPEAN PATENT APPLICATION

(11) **EP 3 992 189 A1**
(43) Date of publication of application: **04.05.2022**
(21) Application number: 20831428.6
(22) Date of filing: 24.06.2020
(51) Int. Cl.: C07D 403/10, C07D 487/00, A61K 31/495, A61K 31/47, A61P 35/00

(54) **QUINAZOLINE AND CINNOLINE DERIVATIVES AS DNA-PK INHIBITOR**

(30) Priority: 27.06.2019 CN 201910568738; 23.01.2020 CN 202010076183; 23.03.2020 CN 202010209372
(71) Applicant: Medshine Discovery Inc., Nanjing, Jiangsu 210032 (CN)
(72) Inventor: CHEN, Kevin X, Shanghai 200131 (CN); CHEN, Zhaoguo, Shanghai 200131 (CN); ZHANG, Li, Shanghai 200131 (CN); HU, Boyu, Shanghai 200131 (CN); CHEN, Shuhui, Shanghai 200131 (CN)
(74) Representative: Secerna LLP
(86) International application number: PCT/CN2020/098198
(87) International publication number: WO 2020/259613

(57) **Abstract**

Disclosed in the present invention are a compound as represented by formula (I), and an isomer or pharmaceutically acceptable salt thereof, and disclosed is an application thereof in preparing drugs for treating DNA-PK inhibitor-related diseases.

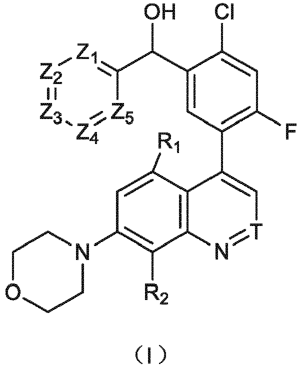

## Description

### The present application claims the following priorities:

CN201910568738.0, filed on June 27, 2019;
CN202010076183.0, filed on January 23, 2020; and
CN202010209372.0, filed on March 23, 2020.

### Technical Field

The present disclosure relates to a series of DNA-PK kinase inhibitors, and use and a synthetic method thereof, and in particular, to use of a compound of formula (I), an isomer thereof or pharmaceutically acceptable salt thereof in the manufacture of a DNA-PK kinase inhibitor related drug.

### Background

DNA Double-Strand Breaks (DSBs), as severe DNA damage, would cause loss of genetic materials and recombination of genes, thereby resulting in cancer or cell death. To maintain gene stability and cell activity, organisms have evolved a DNA Damage Response (DDR) mechanism for damage detection, signal transduction, and damage repair. DNADSB repair mainly includes two types: Homologous Recombination (HR) repair and Non-Homologous End Joining (NHEJ) repair. DDR early damage factors, such as MRN, would detect and identify damage sites, recruit phosphatidylinositol kinase family members (ATM, ATR, and DNA-PK), where phosphorylate H2AX promotes the formation of yH2AX, recruit related signal proteins (such as 53BP1, Chk1, Chk2, BRCA1, and NBS1) to conduct damage signals, so that cells enter the cell cycle arrest state and recruit related repair proteins to repair damaged DNA.

DNA-PK, as an important member of DNA damage repair, mainly targets at the break of non-homologous end joining double strands. It is composed of six core factors: KU70, KU80, DNA-PKCs, CRCC4, ligase IV, and Artemis. When DNA double-strand damage is repaired, KU molecules are specifically connected to the double-strand damage site through a pre-formed channel, recognize and bind ends of the DNA strand, respectively, and then respectively slide along the DNA strand to both ends by a certain distance in an ATP-dependent manner to form KU-DNA complexes that attract DNA-PKcs to the damage site for binding thereto and activating kinase activity, which in turn phosphorylates a series of proteins involved in repair and damage transduction to complete the repair.

At present, induction of DNA damage by radiotherapy and chemotherapy (topoisomerase II, bleomycin, adriamycin, and etoposide) is one of the main means to control tumor growth. However, studies have shown that DNA-PK in tumor tissues after radiotherapy is highly expressed and continuously repairs the tumor cells damaged by radiotherapy and chemotherapy, which has become one of main reasons for drug resistance to radiotherapy and chemotherapy.

The present disclosure aims to discover a DNA-PK small molecule inhibitor, which can inhibit DNA-PK activity by combining with chemoradiotherapy drugs, thereby greatly reducing tumor DNA repair and inducing cell entry into the apoptotic process. This can overcome the problem of drug resistance to radiotherapy and chemotherapy to a large extent and enhance the inhibitory effect on small-cell lung cancer, head and neck cancer, colorectal cancer, pancreatic cancer, and other tumors. This kind of compounds have good activity, show excellent effects and functions, and have a broad prospect.

Patent WO2018178133 discloses a compound M3814, which is a DNA-PK kinase inhibitor, is an antitumor small molecule compound with the following structural formula:

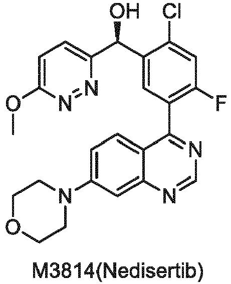

### Content of the present invention

The present disclosure provides a compound of formula (I), an isomer thereof or a pharmaceutically acceptable salt thereof, 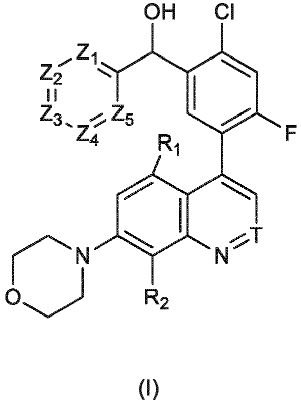 wherein,
T is CH, CR₃ or N;
Z₁, Z₂, Z₃, Z₄, and Z₅ are separately independently N or CR₄;
R₁ and R₂ are separately independently H, F, Cl, Br, I, OH or NH₂;
R₃ is F, Cl, Br, I, OH, NH₂, CN, C₁₋₆ alkyl or C₁₋₆ alkoxy, and the C₁₋₆ alkyl and C₁₋₆ alkoxy are optionally substituted by 1, 2, or 3 Ra;
R₄ is H, F, Cl, Br, I, OH, NH₂, CN, C₁₋₆ alkyl or C₁₋₆ alkoxy, and the C₁₋₆ alkyl and C₁₋₆ alkoxy are optionally substituted by 1, 2, or 3 Rb;
Ra and R_{b} are separately independently F, Cl, Br, I, OH and NH₂,
where when T is CH and R₂ is F, then R₁ is F, Cl, Br, I, OH or NH₂.

In some embodiments of the present disclosure, R₁ and R₂ are separately independently H, Cl, or F, and other variables are as defined in the present disclosure.

In some embodiments of the present disclosure, R₃ is F, Cl, Br, I, OH, NH₂, CN, C₁₋₃ alkyl or C₁₋₃ alkoxy, and the C₁₋₃ alkyl and C₁₋₃ alkoxy are optionally substituted by 1, 2, or 3 Ra, and other variables are as defined in the present disclosure.

In some embodiments of the present disclosure, R₃ is F, Cl, Br, I, OH, NH₂, CN, CH₃, CH₂CH₃ or OCH₃, and the CH₃, CH₂CH₃ and OCH₃ are optionally substituted by 1, 2, or 3 Ra, and other variables are as defined in the present disclosure.

In some embodiments of the present disclosure, R₃ is F, Cl, Br, I, OH, NH₂, CN, CH₃, CH₂F, CHF₂, CF₃, CH₂CH₃ or OCH₃, and other variables are as defined in the present disclosure.

In some embodiments of the present disclosure, R₄ is H, F, Cl, Br, I, OH, NH₂, CN, C₁₋₃ alkyl or C₁₋₃ alkoxy, and the C₁₋₃ alkyl and C₁₋₃ alkoxy are optionally substituted by 1, 2, or 3 R_{b}, and other variables are as defined in the present disclosure.

In some embodiments of the present disclosure, R₄ is H, F, Cl, Br, I, OH, NH₂, CN, CH₃, CH₂CH₃ or OCH₃.

In some embodiments of the present disclosure, T is CH, N, C(NH₂), C(OCH₃) or C(CHF₂), and other variables are as defined in the present disclosure.

In some embodiments of the present disclosure, Z₁, Z₂, Z₃, Z₄, and Z₅ are separately independently N, CH, C(OCH₃) or C(CH₃), and other variables are as defined in the present disclosure.

In some embodiments of the present disclosure, the moiety 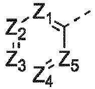 is 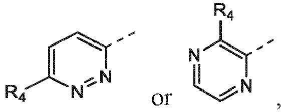 and other variables are as defined in the present disclosure.

In some embodiments of the present disclosure, the moiety 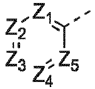 is 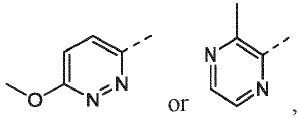 and other variables are as defined in the present disclosure.

In some embodiments of the present disclosure, the moiety 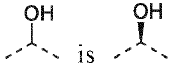 or 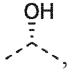 and other variables are as defined in the present disclosure.

Some embodiments further included in the present application are obtained by arbitrarily combining the variables.

In some embodiments of the present disclosure, the compound, the isomer thereof or the pharmaceutically acceptable salt thereof are selected from: 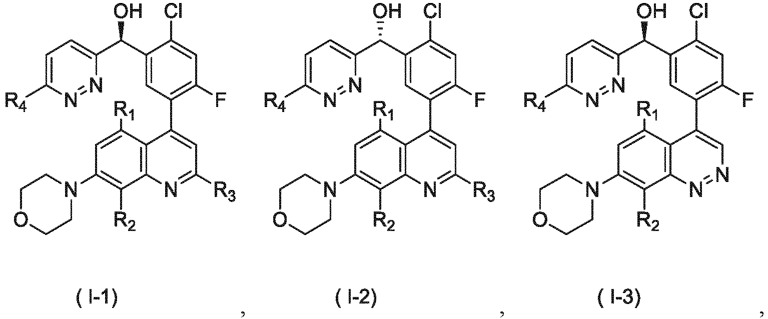 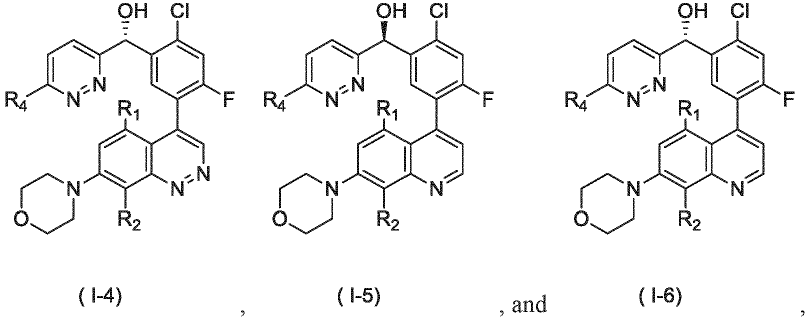 wherein,
R₁, R₂, R₃, and R₄ are as defined in the present disclosure.

The present disclosure further provides a compound of the following formula or a pharmaceutically acceptable salt thereof, which is selected from

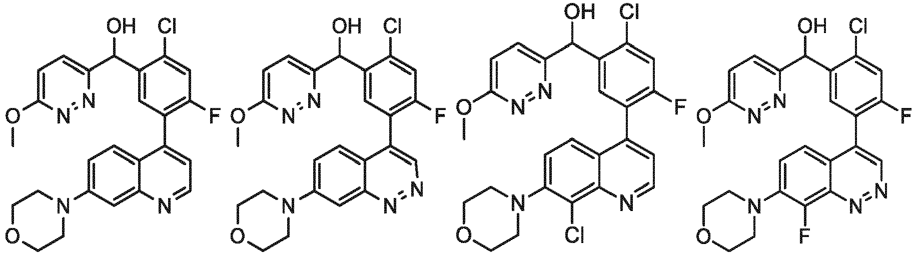

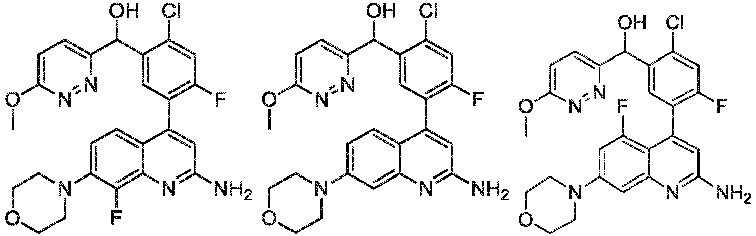

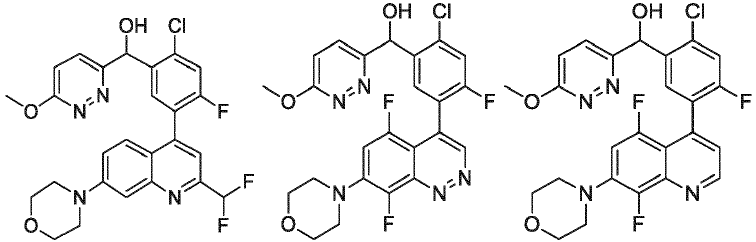

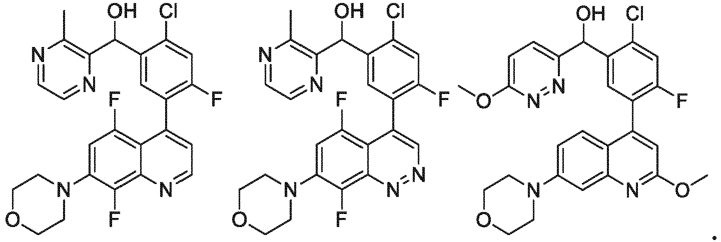

In some embodiments of the present disclosure, the compound or the pharmaceutically acceptable salt thereof is selected from:

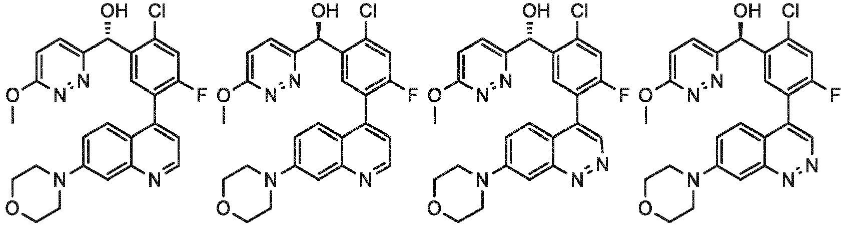

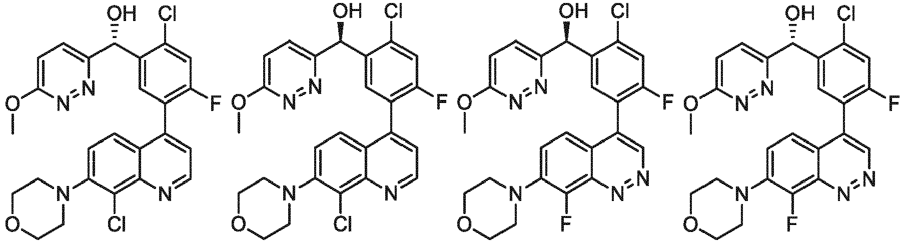

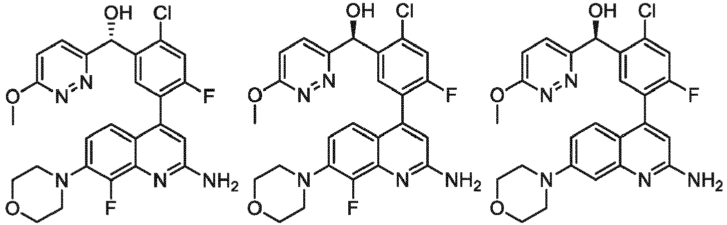

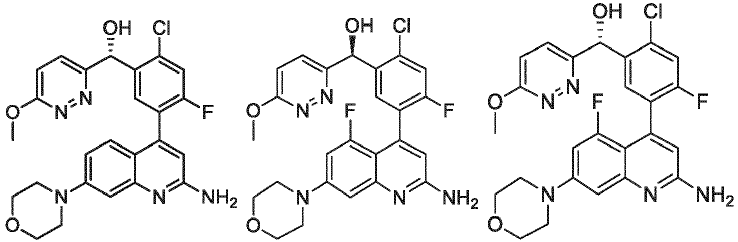

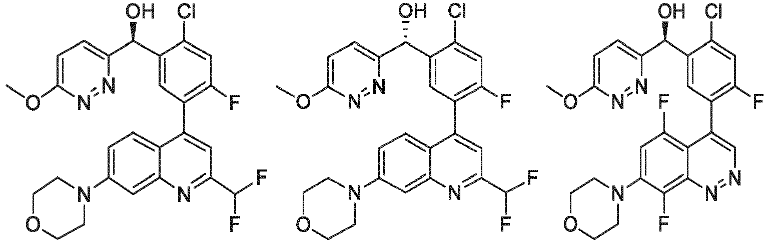

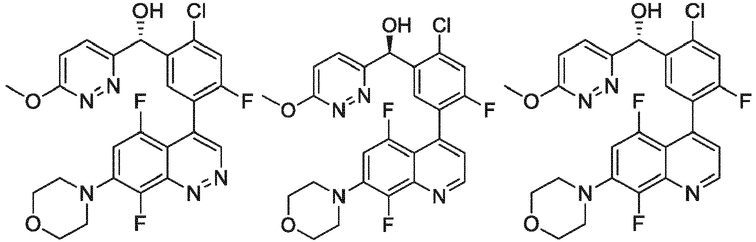

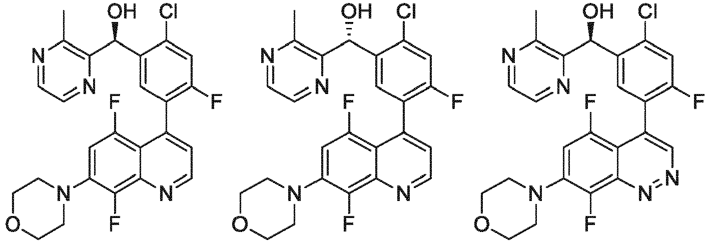

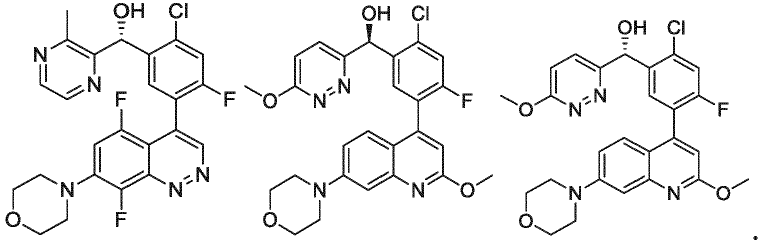

The present disclosure further provides a pharmaceutical composition, comprising a therapeutically effective amount of the compound, the pharmaceutically acceptable salt thereof or the isomer thereof as active ingredients, and a pharmaceutically acceptable carrier.

The present disclosure further provides use of the compound, the isomer thereof or the pharmaceutically acceptable salt thereof in the manufacture of a drug for treating DNA-PK related diseases.

In some embodiments of the present disclosure, the DNA-PK related drug is a drug for treating tumors.

### Technical effect

All compounds of the present disclosure have been tested for DNA-PK kinase activity, and data shows that the activity of some compounds is superior to or equal to the current clinical compound M3814. PK results show that the pharmacokinetic properties of some compounds of the present disclosure are better than those of M3814, and these compounds are good molecules capable of being developed for oral administration. In a transplanted tumor model of NCI-H69 small cell lung cancer, as combined with etoposide (10 mpk), a chemotherapy drug, the synergistic effect is obvious, and as compared with M3814, it shows a considerable tumor suppressive effect and higher safety. In a transplanted tumor model of FaDu head and neck cancer, when combined with radiotherapy, the compound can make the tumor completely disappear, and there is no rebound after drug withdrawal. The compound of the present disclosure has the potential to become an inhibitor for a variety of tumors.

### Definition and explanation

Unless otherwise noted, the following terms and phrases used herein are intended to have the following meanings. One specific term or phrase should not be considered as uncertain or unclear without a specific definition, but should be understood according to an ordinary meaning. When a trade name appears herein, it is intended to refer to the corresponding commodity or its active ingredient.

The term "pharmaceutically acceptable" is used herein for those compounds, materials, compositions and/or dosage forms. They are in a reliable medical judgment range and are adapted to usage in contact with human or animal tissues without excessive toxicity, irritation, anaphylaxis, or other problems or complications, which are commensurate with a reasonable benefit/risk ratio.

The term "pharmaceutically acceptable salt" refers to a salt of the compound of the present application, and is prepared using the compound having a specific substituent discovered in the present application and a relatively nontoxic acid or base. When the compounds of the present disclosure contain relatively acidic functional groups, alkali addition salts can be obtained by contacting such compounds with a sufficient amount of base in a pure solution or a suitable inert solvent. The pharmaceutically acceptable alkali addition salts include sodium, potassium, calcium, ammonium, organic amine or magnesium salts or similar salts. When the compounds of the present disclosure contain relatively alkaline functional groups, acid addition salts can be obtained by contacting such compounds with a sufficient amount of acid in a pure solution or a suitable inert solvent. Examples of pharmaceutically acceptable acid addition salts include inorganic acid salts, organic acid salts, salts of amino acids (such as arginine), and salts of organic acids such as glucuronic acids, where the inorganic acids include, for example, hydrochloric acid, hydrobromic acid, nitric acid, carbonic acid, bicarbonate, phosphoric acid, mono-hydrogen phosphate, dihydrogen phosphate, sulfuric acid, hydrogen sulfate, hydrogen iodate acid, and phosphorous acid; the organic acid salts include, for example, acetic acid, propionic acid, isobutyric acid, maleic acid, malonic acid, benzoic acid, succinic acid, octanedioic acid, fumaric acid, lactic acid, mandelic acid, phthalic acid, benzene sulfonic acid, p-toluenesulfonic acid, citric acid, tartaric acid and mesonic acid and similar acids. Certain specific compounds of the present disclosure contain basic and acidic functional groups, so that the compounds can be converted to any base or acid addition salt.

The pharmaceutically acceptable salt of the present disclosure may be synthesized by using a conventional chemical method from a parent compound containing an acid radical or base. Such salts are generally prepared by reacting these compounds in the form of free acids or bases with stoichiometric appropriate bases or acids in water or an organic solvent or a mixture of both.

Compounds of the present disclosure may have specific geometric or stereoisomer forms. In the present disclosure, it is assumed that all of these compounds, including cis and trans isomers, (-) - and (+) - enantiomers, (R) - and (*S*) - enantiomers, diastereomer, (D) - isomer, (*L*) - isomer, and racemic mixture and other mixtures, for example, a mixture of the enantiomorphism isomers or enrichment of enantiomers. All such mixtures fall within the scope of the present disclosure. Additional asymmetric carbon atoms may be present in alkyl and other substituents. All such isomers and their mixtures are included in the scope of the present disclosure.

Unless otherwise noted, the term "enantiomer" or "optical isomer" refers to stereoisomers that are mirror images of each other.

Unless otherwise noted, the term "cis-trans isomer" or "geometric isomer" arises from the fact that the double bond or single bond of a ring carbon atom cannot rotate freely.

Unless otherwise noted, the term "diastereomer" refers to a stereoisomer in which a molecule has two or more chiral centershttp://baike.baidu.com/item/chiralcenter (http://baike.baidu.com/item/ 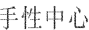 ) and the relationship between the molecules is a non-mirror-image relationshiphttp://baike.baidu.com/item/mirrorimage/19153202 (http://baike.baidu.com/item/ 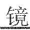 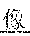 /19153202)http://baike.baidu.com/item/molecule/479055 (http://baike.baidu.eom/item/ 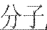 /479055).

Unless otherwise noted, "(+)" represents dextrorotation, "(-)" represents levorotation, and "(±)" represents racemization.

Unless otherwise noted, a wedge solid line bond ( 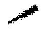 ) and a wedge dashed line bond ( 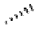 ) represent an absolute configuration of a stereocenter; a straight solid line bond ( 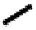 ) and a straight dashed line bond ( 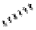 ) represent a relative configuration of the stereocenter; a wavy line ( 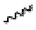 ) represents a wedge solid line bond ( 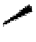 ) or a wedge dashed line bond ( 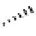 ), or a wavy line ( 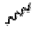 ) represents a straight solid line bond ( 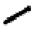 ) and a straight dashed line bond ( 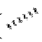 ).

Unless otherwise noted, when the compound has the double bond structure, such as carbon-carbon double bond, carbon-nitrogen double bond, and nitrogen-nitrogen double bond, and each atom on the double bond is connected to two different substituents (in the double bond including nitrogen atoms, a pair of lone pair electrons on the nitrogen atom is considered as a substituent connected thereto); if the atoms on the double bond of the compound is connected to its substituent ( 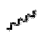 ) with wavy lines, it represents the (Z) type isomer, (E) type isomer or a mixture of the two isomers of the compound. For example, formula (A) below represents that the compound exists as a single isomer of formula (A-1) or (A-2) or as a mixture of the two isomers of formulas (A-1) and (A-2); formula (B) below represents that the compound exists as a single isomer of formula (B-1) or (B-2) or as a mixture of the two isomers of formulas (B-1) and (B-2). Formula (C) below represents that the compound exists as a single isomer of formula (C-1) or (C-2) or as a mixture of the two isomers of formulas (C-1) and (C-2).

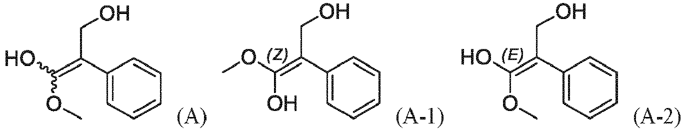

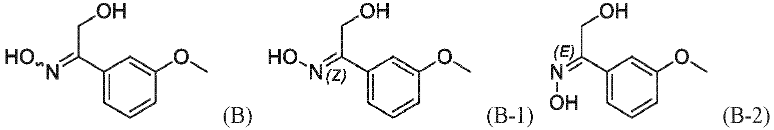

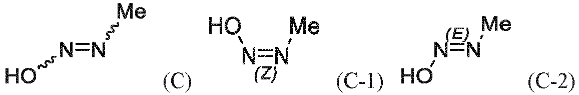

Unless otherwise noted, the term "tautomer" or "tautomeric form" refers to that, at room temperature, different functional group isomers are in dynamic equilibrium and can quickly convert to each other. Chemical equilibrium of tautomers can be achieved if tautomers are possible (for example, in a solution). For example, proton tautomers (also referred to as prototropic tautomers) include mutual transformations through proton transfer, such as keto-enol isomerization and imine-enamine isomerization. Valence tautomers include mutual conversion made by recombination of some bonding electrons. A specific example of keto-enol isomerization is mutual exchange between two tautomers of pentane-2,4-dione and 4-hydroxy-pentane-3 -ene-2-one.

Unless otherwise noted, the term "rich in an isomer", "isomer enrichment", "rich in a kind of enantiomers," or "enantiomer enrichment" indicates that one of the isomer or enantiomer content is less than 100%, and the isomer or enantiomer content is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise noted, the term "isomer excess" or "enantiomer excess" refers to the difference between two isomers or the relative percentage of the two enantiomers. For example, if the content of one isomer or enantiomer is 90% and the other isomer or enantiomer is 10%, the isomer or enantiomer excess (the ee value) is 80%.

Optically active (R) - and (*S*) - isomers as well as *D* and *L* isomers can be prepared by chiral synthesis or chiral reagents or other conventional techniques. If one enantiomer of a certain compound of the present disclosure is desired, it can be prepared by asymmetric synthesis or derivation with a chiral auxiliary agent, in which the resulting diastereomer mixture is separated and the auxiliary group is split to provide the pure desired enantiomer. Alternatively, when the molecule contains basic functional groups (e.g., amino) or acidic functional groups (e.g., carboxyl), salts of diastereoisomers are formed with the appropriate optically-active acids or bases, and the diastereoisomers are then separated by using conventional methods well-known in the art so as to obtain the pure enantiomer by recover0y. In addition, separation of enantiomers and diastereomers is usually accomplished by the use of chromatography using chiral stationary phases, optionally combined with chemical derivations (e.g., the formation of carbamate from amines).

The compound of the present disclosure may include atomic isotopes in unnatural proportions on one or more atoms constituting the compound. For example, radioactive isotopes can be used for labeling the compound, such as tritium (³H), iodine-125 (¹²⁵I), or C-14 (¹⁴C). For another example, deuterium drugs can be formed by replacing hydrogen by heavy hydrogen. Bonds constituted by deuterium and carbon bonds are stronger than those formed by ordinary hydrogen and carbon. Compared with undeuterated drugs, deuterium drugs have advantages of reducing toxic and side effects, increasing drug stability, enhancing efficacy, and prolongating biological half-life of drugs. All transformations of the isotopic composition of compounds of the present disclosure, no matter whether radioactive or not, fall within the scope of the present disclosure.

The term "optional" or "optionally" means that a subsequently described event or condition may, but is not required to, occur, and the description includes the circumstances in which the event or condition occurs and the circumstances in which the event or condition does not occur.

The term "substituted" refers to the substitution of any one or more hydrogen atoms on a particular atom by a substituent group, which may include both heavy hydrogen and hydrogen variants as long as the valence state of the particular atom is normal and the substituted compound is stable. When the substituent is oxygen (i.e., =O), it means that two hydrogen atoms are substituted. Oxygen substitution does not occur on aromatic groups. The term "optionally substituted" means that it may or may not be substituted, except as otherwise specified, and that the type and number of substituents may be arbitrary on the basis of what is chemically achievable.

When any variable (for example, R) occurs once or above in the composition or structure of a compound, its definition in each case is independent. Therefore, for example, if a group is substituted by 0-2 Rs, the group may optionally be substituted by at most two Rs, and R in each case has independent options. In addition, combinations of substituents and/or their variants are permissible only if such combinations produce stable compounds.

When the number of linking groups is zero, for example, -(CRR)₀-, it means that the linking group is a single bond.

When one of the variables is chosen from a single bond, it means that the two groups connected to the single bond are directly connected. For example, when L represents single bond in A-L-Z, it means that the structure is actually A-Z.

When a substituent is vacant, it means that the substituent does not exist. For example, when X is vacant in A-X, it means that the structure is actually A. When the listed substituents do not specify by which atom they are attached to the substituted group, such substituents may be bonded by any atom thereof; for example, a pyridine group may be connected to the substituted group by any of the carbon atoms on the pyridine ring.

Unless otherwise specified, when a group has one or more connectible sites, any one or more sites of the group can be connected to other groups by chemical bonds. When the bonding mode of the chemical bond is not located and there are H atoms at the site that can be connected, the number of H atoms at the site will be correspondingly reduced to groups with corresponding valence numbers with the number of chemical bonds being connected when connecting the chemical bonds. The chemical bonds connected between the site and other groups can be represented by straight solid line bonds ( 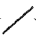 ), straight dashed line bonds ( 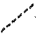 ), or wavy lines ( 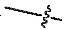 ). For example, the straight solid line bonds in -OCH3 indicate that oxygen atoms in the group are connected to other groups. The straight dashed line bonds in 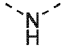 indicate that two ends of the nitrogen atom in the group are connected to other groups. The wavy lines in 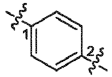 indicate that 1 and 2 position carbon atoms in the phenyl group are connected to other groups. 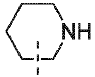 represents that any connectible site on the piperidine base can be connected to other groups by 1 chemical bond, at least including four connection modes: 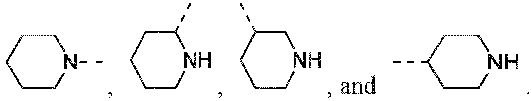 Even if H atoms are drawn on -N-, 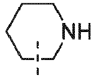 still includes the group of this connection mode 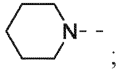 only when connected to one chemical bond, H of the site would be reduced by one so as to become a corresponding univalency piperidine base.

Unless otherwise specified, the term "C₁₋₆ alkyl" is used for representing a saturated hydrocarbon group with a straight-link or branched-link consisting of 1 to 6 carbon atoms. The C₁₋₆ alkyl includes C₁₋₅, C₁₋₄, C₁₋₃, C₁₋₂, C₂₋₆, C₂₋₄, C₆, C₅, and other alkyls, which may be univalent (as in methyl), divalent (as in methylene), or polyvalent (as in hypomethyl). Examples of C₁₋₆ alkyl include, but are not limited to, methyl (Me), ethyl (Et), propyl (including *n*-propyl and isopropyl), butyl (including *n*-butyl, isobutyl, *s*-butyl and *t*-butyl), amyl (including *n*-amyl, iso-amyl and neopentyl), hexyl, etc.

Unless otherwise specified, the term "C₁₋₃ alkyl" is used for representing a saturated hydrocarbon group with a straight-link or branched-link consisting of 1 to 3 carbon atoms. The C₁₋₃ alkyl includes C₁₋₂, C₂₋₃, and other alkyls, which may be univalent (as in methyl), divalent (as in methylene), or polyvalent (as in hypomethyl). Examples of C₁₋₃ alkyl include, but are not limited to, methyl (Me), ethyl (Et), propyl (including *n*-propyl and isopropyl), etc.

Unless otherwise specified, the term "C₁₋₆ alkoxy" is used for representing an alkyl group including 1 to 6 carbon atoms with one oxygen atom connected to the rest part of the molecule. The C₁₋₆ alkoxy includes C₁₋₄, C₁₋₃, C₁₋₂, C₂₋₆, C₂₋₄, C₆, C₅, C₄, C₃ alkoxy, etc. Examples of C₁₋₆ alkoxy include, but are not limited to, methoxy, ethoxy, propoxy (including n-propoxy and isopropoxy), butoxy (including *n*-butoxy, isobutoxy, s-butoxy and *t*-butoxy), pentoxy (including *n*-pentoxy, isopentyl and neopentyl), hexoxy, etc.

Unless otherwise specified, the term "C₁₋₃ alkoxy" is used for representing an alkyl group including 1 to 3 carbon atoms with one oxygen atom connected to the rest part of the molecule. The C₁₋₃ alkoxy includes C₁₋₂, C₂₋₃, C₃, C₂ alkoxy, etc. Examples of C₁₋₃ alkoxy include, but are not limited to, methoxy, ethoxy, propoxy (including n-propoxy and isopropoxy), etc.

Unless otherwise specified, Cₙ₋ₙ₊ₘ or Cₙ-Cₙ₊ₘ includes any one specific situation of n to n+m carbons, for example, C₁₋₁₂ includes C₁, C₂, C₃, C₄, C₅, C₆, C₇, C₈, C₉, C₁₀, C₁₁, and C₁₂, and may also include any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, and the like. Similarly, n member to n+m member represents the number of atoms on the ring is n to n+m, for example, 3-12-member rings include a 3-member ring, a 4-member ring, a 5-member ring, a 6-member ring, a 7-member ring, a 8-member ring, a 9-member ring, a 10-member ring, a 11-member ring, a 12-member ring, and may also include any range from n to n+m, for example, the 3-12-member rings include 3-6-member rings, 3-9-member rings, 5-6-member rings, 5-7-member rings, 6-7-member rings, 6-8-member rings, 6-10-member rings, and the like.

The term "leaving group" refers to a functional group or atom that can be replaced by another functional group or atom through a substitution reaction (for example, a nucleophilic substitution reaction). For example, representative leaving groups include trifluoromethanesulfonic ester; chlorine, bromine, iodine; sulfonic ester groups, such as methylsulfonate, toluene sulfonate, p-bromobenzene sulfonate, and p-toluene sulfonate; and acyl groups, such as acetyl and trifluoroacetyl.

The term "protective group" includes, but is not limited to, "amino protective group", "hydroxyl protective group" or "sulfydryl protective group". The term "amino protective group" refers to a protective group suitable for preventing side reactions at the amino nitrogen position. Representative amino protective groups include, but are not limited to: formyl; acyl groups, for example, chain alkyyl groups (e.g., acetyl, trichloroacetyl or trifluoroacetyl); alkoxy carbonyl, for example, tert-butylcarbonyl (Boc); aryl methoxycarbonyl, such as benzyl methoxycarbonyl (Cbz) and 9-fluorene methoxycarbonyl (Fmoc); aryl methyl groups, such as benzyl (Bn), triphenylmethyl (Tr), 1,1-bis-(4'-methoxy phenyl) methyl; and methylsiliconyl, such as trimethylsiliconyl (TMS) and tert-butyl dimethylsiliconyl (TBS). The term "hydroxyl protective group" refers to a protective group suitable for preventing hydroxyl side reactions. Representative hydroxyl protective groups include, but are not limited to: alkyl, such as methyl, ethyl, and tert-butyl; acyl groups, for example, chain alkyl groups (e.g., acetyl groups); aryl methyl groups, such as benzyl (Bn), p-methoxy benzyl (PMB), 9-fluorenyl methyl (Fm), and diphenylmethyl (DPM); methylsiliconyl, such as trimethylsiliconyl (TMS) and tert-butyl dimethylsiliconyl (TBS).

Compounds of the present disclosure can be prepared by using a variety of synthetic methods well-known by a person skilled in the art, including the following listed specific implementations, implementations formed by the combination of same and other chemical synthesis methods, and equivalent replacement modes well-known to a person skilled in the art; and the preferred implementations include, but are not limited to, the embodiments of the present disclosure.

The structure of the compound of the present disclosure can be confirmed by using conventional methods well-known to a person skilled in the art. If the present disclosure relates to an absolute configuration of the compound, the absolute configuration can be confirmed by conventional technical means in the art. For example, Single Crystal X-Ray Diffraction (SXRD) relates to, diffraction intensity data of a cultured single crystal is collected with Bruker D8 Venture diffrometer; a light source is CuKα radiation, and a scanning mode is ϕ/ω scanning. After relevant data is collected, a direct method (Shelxs97) is further adopted to analyze a crystal structure, so that an absolute configuration could be confirmed.

The solvent used in the present disclosure is commercially available. The present disclosure adopts the following abbreviations: aq represents water; HATU represents O-(7-azobenzotriazol-1-yl)-N,N,N',N'-tetramethylurea-hexafluorophosphate; EDC represents N-(3-dimethylaminopropyl)-N'-ethyl carbon diimine hydrochloride; m-CPBA represents 3-chloroperoxybenzoic acid; eq represents equivalent and equal quantity; CDI represents carbonyl diimidazole; DCM represents dichloromethane; PE represents petroleum ether; DIAD represents diisopropyl azo dicarboxylate; DMF represents N,N-dimethylformamide; DMSO represents dimethyl sulfoxide; EtOAc represents ethyl acetate; EtOH represents ethanol; MeOH represents methanol; CBz represents benzoxy carbonyl group, which is an amine protective group; BOC represents tert-butylcarbonyl, which is an amine protective group; HOAc represents acetic acid; NaCNBH₃ represents sodium cyanoborohydride; r.t. represents room temperature; O/N represents overnight; THF represents tetrahydrofuran; Boc₂O represents di-tert-butyl dicarbonate; TFA represents trifluoroacetic acid; DIPEA represents diisopropyl ethyl amine; SOCl₂ represents sulfoxide chloride; CS₂ represents carbon disulfide; TsOH represents p-toluene sulfonic acid; NFSI represents N-fluoro-N-(benzensulfonyl) benzensulfonamide; NCS represents 1-chloropyrrolidene-2,5-dione; *n*-Bu₄NF represents tetrabutylammonium fluoride; iPrOH represents 2-propanol; mp represents melting point; LDA represents diisopropylamine lithium; Pd₂(dba)₃ represents tri(dibenzylidenyl acetone)dipalladium; PO represents oral administration; QD represents once a day; IP represents intraperitoneal injection; BID means twice a day; and Gy represents the unit of measurement of radiotherapy dose is gray.

Compounds are named according to general naming principles in the art or by using ChemDraw^{®} software, and commercially available compounds are named by the supplier catalog.

### Detailed description of the preferred embodiment

The present disclosure is described in detail through embodiments below, but it does not mean any unfavorable restriction is imposed on the present disclosure. The present application has been described in detail herein, and specific embodiment modes are also disclosed. For a person skilled in the art, it would be obvious to make various changes and improvements to the detailed description of the present disclosure without departing from spirits and scope of the present disclosure.

### Embodiments 1 & 2

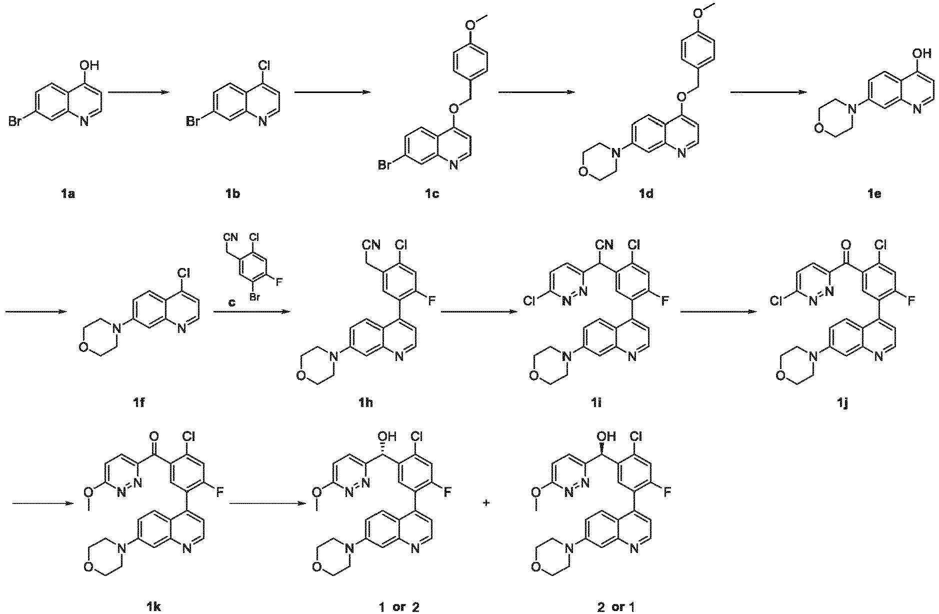

### Step I

A compound 1a (5 g, 22.3 mmol, 1 eq) was added into phosphorus oxychloride (18.6 g, 121 mmol, 11.2 mL, 5.42 eq); a mixture was stirred for 2 hours in a dry condition at 110°C; after the reaction was completed, the mixture was slowly dropped into a saturated potassium carbonate solution (300 mL) for dilution, extracted with ethyl acetate (50 mL^{∗}3) , and the combined organic matter was dried with anhydrous sodium sulfate and filtered to obtain a compound 1b.

### Step II

P-methoxybenzyl alcohol (5.70 g, 41.2 mmol, 5.13 mL, 2 eq) was dissolved into *N,N-*dimethylformamide (50.0 mL); a mixture was stirred at 0°C and sodium hydride (1.65 g, 41.2 mmol, 60% purity, 2 eq) was slowly added; and the reaction was continued at this temperature for 0.5 hour. Then the compound 1b (5.00 g, 20.6 mmol, 1 eq) was further added; the mixture was stirred at 80°C for 4.5 h; after the reaction was completed, water (250 mL) was added for dilution and filtering, and after a filter cake was decompressed and concentrated, a compound 1c was obtained.

MS-ESI calculation value [M+H]⁺ 344, 346; actual measurement value 344, 346.

### Step III

The compound 1c (6 g, 17.4 mmol, 1 eq), morpholine (7.59 g, 87.2 mmol, 7.67 mL, 5 eq), sodium tert-butoxide (3.35 g, 34.9 mmol, 2 eq), tri(dibenzylacetone)dipalladium (798 mg, 872 µmol, 0.05 eq), and (±)-2,2-bis(diphenylphosphonyl)-11-binaphthalene (542 mg, 872 µmol, 0.05 eq) were added into methylbenzene (60 mL); and the mixture was stirred at 100°C for 3 hours under the protection of nitrogen. After the reaction was completed, decompression and concentration were performed; water (80 mL) was added for dilution; ethyl acetate (50 mL ^{∗} 3) extraction was performed; and column chromatography was conducted to obtain a compound 1d.

¹H NMR (400 MHz, DMSO-*d*₆) δ:8.57 (d, *J*=5.2 Hz, 1H), 7.95 (d, *J*=9.2 Hz, 1H), 7.47 (d, *J*=8.4 Hz, 2H), 7.36 (dd, *J*=2.0, 9.2 Hz, 1H), 7.17 (d, *J*=2.0 Hz, 1H), 6.98 (d, *J*=8.4 Hz, 2H), 6.89 (d, J=5.2 Hz, 1H), 5.25 (s, 2H), 3.78 (br s, 4H), 3.34 (s, 3H), 3.31-3.20 (m, 4H).

### Step IV

The compound 1d (5.2 g, 14.9 mmol, 1 *eq)* was dissolved into trifluoroacetic acid (20.0 mL); the mixture was stirred at 80°C for 20 min. After the reaction was completed, and after decompression and concentration were performed; pulped with petroleum ether: ethyl acetate was 20:1; filtered and dried to obtain compound 1e.

MS-ESI calculation value [M+H]⁺ 231; actual measurement value 231.

¹H NMR (400 MHz, DMSO-*d*₆) δ :13.58 (br s, 1H), 8.40 (d, *J*=6.8 Hz, 1H), 8.06 (d, *J*=9.2 Hz, 1H), 7.45 (dd, *J*=2.0, 9.2 Hz, 1H), 7.01 (d, *J*=2.0 Hz, 1H), 6.68 (d, *J*=7.2 Hz, 1H), 3.82-3.74 (m, 4H), 3.34-3.42 (m, 4H).

### Step V

The compound 1e(6 g, 17.4 mmol, 1 eq) was added into phosphorus oxychloride (45.5 g, 297 mmol, 27.6 mL, 17.0 eq); the mixture was stirred for 1 hour in a dry condition at 110°C; after the reaction was completed, most of phosphorus oxychloride was removed through decompression and concentration; the remaining mixture was slowly dropped into a saturated potassium carbonate solution (200 mL) for dilution; ethyl acetate extraction (50.0 mL^{∗}3) was performed; combined organic matter was dried with the anhydrous sodium sulfate and filtered; and upon decompression and concentration, pulping was performed by using petroleum ether and ethyl acetate at a ratio of 20:1 to obtain a compound 1f.

MS-ESI calculation values [M+H]⁺ 249, 251; actual measurement values 249, 251.

¹H NMR (400 MHz, DMSO-*d*₆) δ: 8.66 (d, *J*=4.8 Hz, 1H), 8.01 (d, *J*=9.2 Hz, 1H), 7.61 (dd, *J*=2.4, 9.6 Hz, 1H), 7.44 (d, *J*=4.8 Hz, 1H), 7.29 (d, *J*=2.4 Hz, 1H), 3.76-3.82 (m, 4H), 3.34 (br s, 4H).

### Step VI

A compound c (2.60 g, 10.5 mmol, 1 eq) and double-pinacol alcohol boric acid ester (3.19 g, 12.6 mmol, 1.2 eq) were added into anhydrous dioxane (20.0 mL), and then potassium acetate (2.05 g, 20.9 mmol, 2 eq) and dichloride(triphenylphosphine)palladium (II) (367 mg, 523 µmol, 0.05 eq) were added and stirred at 130° for 0.5 h under the protection of nitrogen; the compound 1f (1.56 g, 6.28 mmol, 0.6 eq), 1,1-bis(diphenyl phosphorus) ferrocene palladium chloride (383 mg, 523 µmol, 0.05 eq), sodium carbonate (2.22 g, 20.9 mmol, 2 eq), and water (4.00 mL) were then added and stirred at 90° for 2 h under the protection of nitrogen; after the reaction was completed, most of dioxane was removed by decompression and concentration; after water was added for dilution, ethyl acetate extraction was conducted (50.0 mL^{∗}3); the combined organic matter was dried with anhydrous sodium sulfate, filtered, decompressed and concentrated, and column chromatography purification was performed to obtain a compound 1h.

MS-ESI calculation values [M+H]⁺ 382, 384; actual measurement values 382, 384.

### Step VII

The compound 1h (1.8 g, 4.71 mmol, 1 eq) was dissolved into dimethylsulfoxide (20.0 mL) and then potassium hydroxide (688 mg, 12.3 mmol, 2.6 eq) and 3,6-dichloropyridazine (632 mg, 4.24 mmol, 0.9 eq) were further added for reaction for 1 hour at 40°C; after the reaction was completed, and after water (30.0 mL) was added for dilution, ethyl acetate extraction (50.0 mL^{∗}3) was performed; combined organic matter was washed with saturated salt solution (20 mL), dried with anhydrous sodium sulfate, and filtered; and upon decompression spin dry, a compound 1i was obtained.

MS-ESI calculation values [M+H]⁺ 494, 495, 496; actual measurement values 494, 495, 496.

### Step VIII

The compound 1i (1.8 g, 3.64 mmol, 1 eq) was dissolved into acetonitrile (15.0 mL) and then potassium carbonate (688 mg, 12.3 mmol, 2.6 eq) and 30% aqueous hydrogen peroxide solution (2.00 mL) were further added for reaction for 20 min at 20°C; after the reaction was completed, a saturated sodium thiosulfate solution (150 mL) was added at 0°C for cancellation, and then water (50.0 mL) was added for dilution; ethyl acetate extraction (50.0 mL^{∗}3) was performed; combined organic matter was washed with saturated salt solution (20.0 mL), dried with anhydrous sodium sulfate, and filtered; and upon decompression spin dry, a compound 1j was obtained.

MS-ESI calculation values [M+H]⁺ 483, 484, 485; actual measurement values 483, 484, 485.

### Step IX

The compound 1j (1.75 g, 3.62 mmol, 1 eq) was dissolved into methanol (10.0 mL) and then potassium carbonate (1.00 g, 7.24 mmol, 2 eq) was further added for reaction for 30 min at 40°C; after the reaction was completed, water (100 mL) was added for dilution, and then filtering was performed. Upon decompression spin dry of the filter cake, a compound 1k was obtained.

MS-ESI calculation values [M+H]⁺ 479, 481; actual measurement values 479, 481.

### Step X

The compound 1k (1.6 g, 3.34 mmol, 1 eq) was dissolved into methanol (20.0 mL) and then sodium borohydride (1.90 g, 50.1 mmol, 15 eq) was slowly added for reaction for 1 hour at 25°C; after the reaction was completed, most of methanol was removed by decompression and concentration; after water (30.0 mL) was added for dilution, ethyl acetate extraction (50.0 mL^{∗}3) was performed; combined organic matter was washed with saturated salt solution (20.0 mL), dried with anhydrous sodium sulfate and filtered, and decompression and concentration were performed; after column chromatography purification, upon High Performance Liquid Chromatography (HPLC) separation and preparation, and upon chiral separation (chromatographic column: Chiralcel OJ-3 100×4.6mm I.D., 3 µm); mobile phase [A phase: carbon dioxide, B phase: ethanol (containing 0.05% diethylamine); gradient: B phase rising from 5% to 40% in 4.5 min, maintaining at 40% for 2.5 min, and then maintaining B phase at 5% for 1 min]; flow rate: 2.8 mL/min; column temperature: 40°C; elution time: 8 min), compounds 1 (retention time: 3.406 min) and 2 (retention time: 3.913 min) were obtained through separation.

MS-ESI calculation values [M+H]⁺ 481, 483; actual measurement values 481, 483.

### Compound 1 (retention time: 3.406 min)

¹H NMR (400 MHz, DMSO-*d*₆) δ: 8.83 (d, *J*=4.4 Hz, 1H), 7.77 (d, *J*=8.0 Hz, 1H), 7.73-7.62 (m, 2H), 7.47 (br s, 2H), 7.33 (s, 1H), 7.26 (d, *J*=4.4 Hz, 1H), 7.21 (d, *J*=9.2 Hz, 1H), 6.61 (br s, 1H), 6.22 (br s, 1H), 4.00 (s, 3H), 3.76-3.81 (m, 4H), 3.32-3.30 (m, 4H).

### Compound 2 (retention time: 3.913 min)

¹H NMR (400 MHz, DMSO-*d*₆) δ: 8.83 (d, *J*=4.4 Hz, 1H), 7.77 (br d, *J*=8.0 Hz, 1H), 7.74-7.64 (m, 2H), 7.47 (br s, 2H), 7.34 (s, 1H), 7.27 (d, *J*=4.4 Hz, 1H), 7.21 (d, *J*=9.2 Hz, 1H), 6.62 (br s, 1H), 6.23 (br s, 1H), 4.01 (s, 3H), 3.74-3.86 (m, 4H), 3.26-3.32 (m, 4H).

### Embodiments 3 & 4

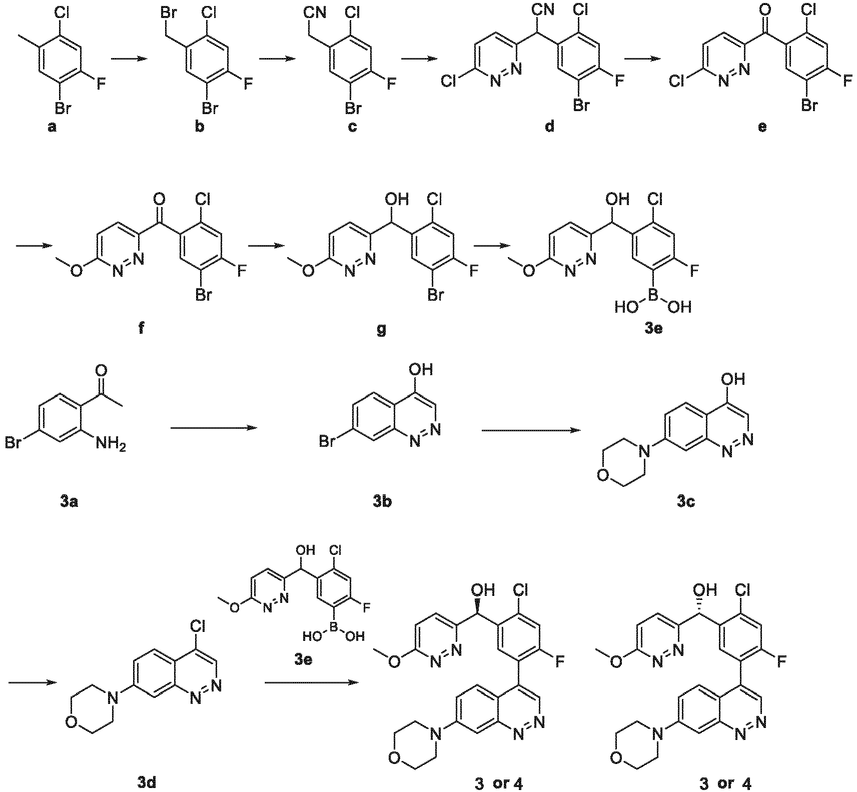

### Step I

A compound a (48 g, 215 mmol, 1.00 *eq*), N-bromosuccinimide (42.1 g, 236 mmol, 1.10 *eq*), and benzoyl peroxide (1.04 g, 4.30 mmol,0.02 *eq*) were dissolved into acetonitrile (80.0 mL) for reaction for 4 h at 90°C; after the reaction was completed, upon decompression spin dry and filtering, ethyl acetate (50.0 mL) was used for washing the filter cake; after the filtrate was diluted by adding saturated salt solution (100 mL), ethyl acetate (200 mL) was used for extraction; the organic matter was washed with saturated salt solution (100 mL) twice, dried with anhydrous sodium sulfate, and filtered; and upon decompression spin dry, a compound b was obtained.

¹H NMR (400MHz, CDCl₃) δ: 7.63 (d, *J*=7.2 Hz, 1H), 7.18 (d, *J*=8.0 Hz, 1H), 4.49 (s, 2H).

### Step II

The compound b (73.4 g, 243 mmol, 1.00 *eq)* was dissolved into *N,N-*dimethylformamide (100 mL) and water (30.0 mL); potassium cyanide (25.3 g, 389 mmol, 1.60 *eq*) in batches were added to the reaction solution for reaction for 2 h at 25°C; after the reaction was completed, water (500 mL) was added for dilution; ethyl acetate (300 mL) was used for extraction; the organic matter was washed with saturated salt solution (150 mL) twice, dried with anhydrous sodium sulfate, and filtered; and upon decompression spin dry, column chromatography was used to obtain a compound c.

¹H NMR (400MHz, CDCl₃) δ: 7.72 (d, *J*=7.2 Hz, 1H), 7.24 (s, 1H), 3.79 (s, 2H).

### Step III

The compound c (10 g, 40.2 mmol, 1.00 *eq)* and 3,6-dichloropyridazine (8.99 g, 60.4 mmol, 1.5 *eq*) were dissolved in dimethylsulfoxide (50.0 mL). Potassium hydroxide (3.39 g, 60.4 mmol, 1.5 *eq*) in batches was added to the reaction solution for reacting at 30°C for 2 hours. After the reaction was completed, water (250 mL) was added for dilution; ethyl acetate (500 mL) extraction was performed; the organic matter was washed with saturated salt solution (400 mL) twice, dried with anhydrous sodium sulfate, and filtered; decompression spin dry was performed; and column chromatography purification was conducted to obtain a compound d.

MS-ESI calculation values [M+H]⁺ 360, 362, 364; actual measurement values 360, 362, 364.

¹H NMR (400MHz, DMSO-*d*₆) δ: 8.00 (s, 1H), 7.97 (s, 1H), 7.84 (d, *J*=4.0 Hz, 1H), 7.82 (d, *J*=4.0 Hz, 1H), 6.49 (s, 1H).

### Step IV

The compound d (6.00 g, 16.6 mmol, 1.00 *eq)* and potassium carbonate (2.99 g, 21.6 mmol, 1.30 *eq)* were dissolved in acetonitrile (18.0 mL). Hydrogen peroxide of 30% mass fraction (6.01 g, 53.0 mmol, 3.19 *eq)* in batches was added to the reaction solution for reacting at 20°C for 20 min. After the reaction was completed, the reaction solution was slowly dropped into a cold saturated sodium thiosulfate solution (15.0 mL); water (20.0 mL) was added for dilution; ethyl acetate (40.0 mL) extraction was performed; the organic matter was washed with saturated salt solution (40.0 mL) twice, dried with anhydrous sodium sulfate, and filtered; and decompression spin dry was performed to obtain a compound e.

MS-ESI calculation values [M+H]⁺ 349, 351, 353; actual measurement values 349, 351, 353.

¹H NMR (400MHz, CDCl₃) δ: 8.19 (d, *J*=8.8 Hz, 1H), 7.81 (d, *J*=7.2 Hz, 1H), 7.76 (d, *J*=8.8 Hz, 1H), 7.28 (s, 1H).

### Step V

The compound e (5.75 g, 16.4 mmol, 1.00 *eq*) and potassium carbonate (2.50 g, 18.1 mmol, 1.10 *eq*) were dissolved in methanol (50.0 mL) for reaction at 20°C for 6 hours. After the reaction was completed, water (200 mL) was added for dilution; filtering was performed; the filter cake was washed with water (40.0 mL) twice; ethyl acetate (300 mL) was used for dilution; anhydrous sodium sulfate was used for drying; filtering was performed; and decompression spin dry was performed to obtain a compound f.

MS-ESI calculation values [M+H]⁺ 345, 347, 349; actual measurement values 345, 347, 349.

¹H NMR (400MHz, CDCl₃) δ: 8.17 (d, *J*=9.2Hz, 1H), 7.73 (d, *J*=7.2Hz, 1H), 7.24 (s, 1H), 7.15 (d, *J*=9.2 Hz, 1H), 4.24 (s, 3H).

### Step VI

The compound f (10.0 g, 28.9 mmol, 1.00 *eq)* was dissolved in methanol (100 mL). Sodium borohydride (1.3 g, 34.4 mmol, 1.19 *eq*) in batches was added to the reaction solution for reacting at 15°C for 1 hour. After the reaction was completed, water (20.0 mL) was added for cancellation; dichloromethane (500 mL) was used for dilution; saturated salt solution (200 mL) was used for washing twice; anhydrous sodium sulfate was used for drying; filtering was performed; decompression spin dry was performed; and column chromatography purification was conducted to obtain a compound g.

MS-ESI calculation values [M+H]⁺ 345, 347, 349; actual measurement values 345, 347, 349.

¹H NMR (400MHz, DMSO-*d*₆) δ:7.95 (d, *J*=7.6 Hz, 1H), 7.70 (d, *J*=8.8 Hz, 1H), 7.65-7.59 (m, 1H), 7.21 (d, *J*=8.8 Hz, 1H), 6.66 (d, *J*=4.8 Hz, 1H), 6.10 (d, *J*=4.8 Hz, 1H), 3.99 (s, 3H).

### Step VII

Pd(PPh₃)₂Cl₂ (41.31 mg, 58.86 µmol, 0.05 eq) and potassium acetate (346.6 mg, 3.53 mmol, 3 eq) were added into a dioxane (20 mL) solution of the compound g (409 mg, 1.18 mmol, 1 eq) and double-pinacol alcohol boric acid ester (448 mg, 1.77 mmol, 1.5 eq). The obtained reaction liquid was stirred at 130°C for 4 hours. The reaction was completed, the reaction solution was concentrated, and the residue was separated and purified by HPLC to obtain a compound 3e.

MS-ESI calculation values [M+H]⁺ 313, 315; actual measurement values 313, 315.

### Step VIII

A compound 3a (288 mg, 1.35 mmol, 1 eq) was added into hydrochloric acid (5.00 mL) and water (1.00 mL); then the aqueous solution (1.00 mL) of sodium nitrite (102 mg, 1.48 mmol, 1.1 eq) was dropped at 0°C for stirring for 30 min at this temperature, and the temperature was risen to 70°C for stirring for 12 hours. After the reaction was completed, water (20.0 mL) was added for dilution and filter. The filter cake was decompressed and concentrated to obtain a compound 3b.

MS-ESI calculation values [M+H]⁺ 225, 227; actual measurement values 225, 227.

¹H NMR (400 MHz, DMSO-*d*₆) δ:13.53 (br s, 1H), 7.95 (d, *J*=8.8 Hz, 1H), 7.80-7.73 (m, 2H), 7.56 (dd, *J*=1.6, 8.7 Hz, 1H).

### Step IX

The compound 3b (70 mg, 311 µmol, 1 eq), morpholine (54.2 mg, 622 µmol, 54.8 µL, 2 eq), mesylate (2-dicyclohexylphosphone-2,6-diisopropoxy-1,1-biphenyl)(2-amino-1,1-biphenyl)palladium (II) (43.8 mg, 52.3 µmol, 1.68e-1 eq), and potassium tert-butoxide (105 mg, 933 µmol, 3 eq) were added into anhydrous tetrahydrofuran (1.00 mL), and the mixture was stirred at 80°C for 12 hours. After the reaction was completed, filtering and decompression and concentration were performed; and column chromatography (20:1 dichloromethane: methanol) purification was performed to obtain a compound 3c.

MS-ESI calculation value [M+H]⁺ 232; actual measurement value 232.

¹H NMR (400 MHz, DMSO-*d*₆) δ:7.84 (d, *J*=9.2 Hz, 1H), 7.57 (s, 1H), 7.20 (br d, *J*=9.2 Hz, 1H), 6.67 (s, 1H), 3.77-3.75 (m, 4H), 3.30-2.29 (m, 4H).

### Step X

The compound 3c (70.0 mg, 303 µmol, 1 eq) was dissolved into phosphorus oxychloride (2.18 mL), and the mixture was reacted at 110°C for 30 min. After the reaction was completed, decompression and concentration were conducted to remove phosphorus oxychloride; anhydrous dioxane (20.0 mL) was further used for dilution; and after decompression and concentration, a compound 3d was obtained.

MS-ESI calculation value [M+H]⁺ 250, 252; actual measurement values 250, 252.

### Step XI

The compound 3e (250 mg, 801 µmol, 2 eq), compound 3d (100 mg, 401 µmol, 1 eq), 1,1-bis(diphenyl phosphorus) ferrocene palladium chloride (23.4 mg, 32.0 µmol, 0.08 eq), and sodium carbonate (127 mg, 1.20 mmol, 3 eq) were added into anhydrous dioxane (5.00 mL) neutralization water (1.00 mL) to be stirred at 80° for 2 h under the protection of nitrogen; after the reaction was completed, most of dioxane was removed by decompression and concentration; after water (20.0 mL) was added for dilution, dichloromethane extraction was conducted (25.0 mL^{∗}2); the combined organic matter was washed by saturated salt solution, dried with anhydrous sodium sulfate, and filtered; and decompression and concentration were conducted. After column chromatography purification, and upon chiral separation (chromatographic column: Chiralpak AD-3 50^{∗}4.6mm I.D., 3 µm); mobile phase [40% ethanol carbon dioxide solution (containing 0.05% diethylamine); flow rate: 4 mL/min; column temperature: 40°C; elution time: 3 min), a compound 3 (retention time: 0.845 min) and a compound 4 (retention time: 1.890 min) were obtained.

MS-ESI calculation value [M+H]⁺ 482, 484; actual measurement value 482, 484.

### Compound 3 (retention time: 0.845 min)

¹H NMR (400 MHz, acetonitrile-d) δ:9.03 (s, 1H), 7.77 (d, *J*=7.8 Hz, 1H), 7.67 (s, 1H), 7.65-7.56 (m, 3H), 7.47 (d, *J*=9.2 Hz, 1H), 7.06 (d, *J*=9.2 Hz, 1H), 6.35 (d, *J*=4.4 Hz, 1H), 4.68 (br d, J=4.4 Hz, 1H), 4.05 (s, 3H), 3.87-3.83 (m, 4H), 3.44-3.39 (m, 4H).

### Compound 4 (retention time: 1.890 min)

¹H NMR (400 MHz, acetonitrile-d) δ:9.04 (s, 1H), 7.77 (d, *J*=8.0 Hz, 1H), 7.67 (s, 1H), 7.64-7.57 (m, 3H), 7.49-7.45 (m, 1H), 7.06 (d, *J*=9.2 Hz, 1H), 6.35 (d, *J*=4.4 Hz, 1H), 4.68 (br s, 1H), 4.05 (s, 3H), 3.87-3.83 (m, 4H), 3.43-3.40 (m, 4H).

### Embodiments 5 & 6

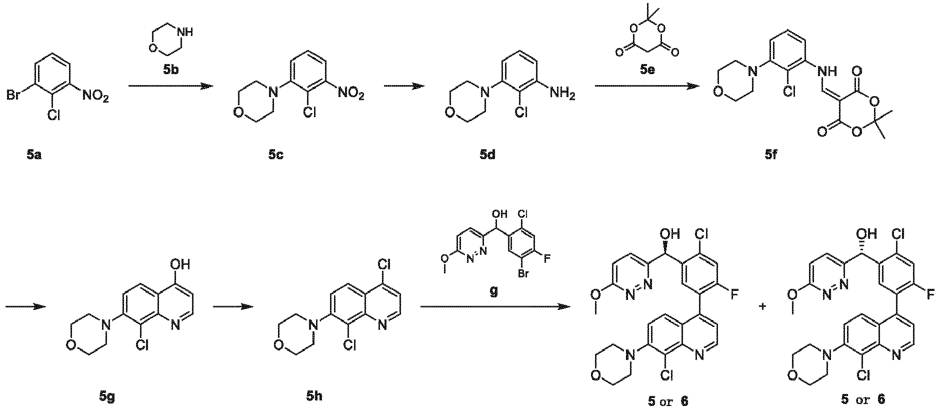

### Step I

A dioxane (40.0 mL) solution of a compound 5a (3.3 g, 14.0 mmol, 1 *eq)* and morpholine (1.22 g, 14.0 mmol, 1.23 mL, 1 *eq*) was added with cesium carbonate (9.09 g, 27.9 mmol, 2 *eq),* Pd₂(dba)₃ (639 mg, 698 µmol, 0.05 *eq*), and 4,5-diphenylphosphone-9,9-dimethyloxanthracene (808 mg, 1.40 mmol, 0.1 eq) and then stirred at 85°C for 16 h under the protection of nitrogen. After decompression and concentration were performed to remove the solvent, 30.0 mL of water was used for dilution, and then 50.0 mL of ethyl acetate was used for extraction. The separated organic matter was washed with saturated salt solution (50.0 mL), dried with anhydrous sodium sulfate, and filtered; and a crude product was obtained through concentration. Column chromatography was conducted to obtain a compound 5c.

MS-ESI calculation value [M+H]⁺ 243, 245; actual measurement values 243, 245.

### Step II

Iron powder (1.24 g, 22.2 mmol, 6 *eq)* and ammonium chloride (1.19 g, 22.3 mmol, 778 µL, 6 *eq*) were added into the solution of ethyl alcohol (10.0 mL) and water (2.00 mL) of the compound 5c (900 mg, 3.71 mmol, 1 *eq),* and the obtained mixture was stirred at 85°C for 2 hours. The filtrate was cooled to the room temperature and then was filtered by kieselguhr and decompressed and concentrated. The residue was diluted by water (50.0 mL); then ethyl acetate (50.0 mL) extraction was performed; the combined organic matter was washed with saturated salt solution (50.0 mL), dried with anhydrous sodium sulfate, and filtered; decompression and concentration were performed to obtain the crude product; and the crude product was subjected to column chromatography to obtain a compound 5d. MS-ESI calculation values [M+H]⁺ 213, 215; actual measurement values 213, 215.

### Step III

A solution of triethyl orthoformate (4.46 g, 30.1 mmol, 5.00 mL, 10.66 *eq)* of the compound 5e (610 mg, 4.23mmol, 1.5 *eq)* was in backflow at 135°C for 1 hour. After cooling to the room temperature (10-20°C), the compound 5d (600 mg, 2.82 mmol, 1 *eq*) was added into the reaction solution and was continuously in backflow at 135°C for 2 hours. Upon decompression and concentration to remove the solvent, a compound 5f was obtained.

MS-ESI calculation value [M+H]⁺ 367, 369; actual measurement values 367, 369.

¹H NMR (400 MHz, DMSO-*d*₆) δ:11.65 (br d, *J*=13.6 Hz, 1 H), 8.74 (d, *J*=13.6 Hz, 1 H), 7.60 (d, *J*=8.0 Hz, 1 H), 7.41 (t, *J*=8.0 Hz, 1 H), 7.11-7.01 (m, 1 H), 3.78-7.738 (m, 4 H), 3.01-2.95 (m, 4 H), 1.69 (s, 6 H).

### Step IV

The compound 5f (1.3 g, 3.54 mmol, 1 *eq*) was heated in a solution of diphenyl diphenyl ether (3.54 mmol, 13.0 mL, 1 *eq*) to 260°C for reacting for 1 hour. The reaction solution was cooled to the room temperature (10-20°C), sediment formed along with cooling was filtered, and petroleum ether (5.00 mL^{∗}3) was used for washing, to obtain a compound 5g

MS-ESI calculation value [M+H]⁺ 265, 268; actual measurement values 265, 268.

¹HNMR (400 MHz, DMSO-*d*₆) δ:11.18 (br d, *J*=5.6 Hz, 1 H), 8.03 (d, *J*=8.8 Hz, 1 H), 7.19 (d, *J*=8.8 Hz, 1 H), 7.01 (d, *J*=7.8 Hz, 1 H), 6.03 (d, *J*=6.8 Hz, 1 H), 3.81-3.76 (m, 4 H), 3.14-3.09 (m, 4 H).

### Step V

A mixture of the compound 5g (0.5 g, 1.89 mmol, 1 *eq)* and phosphorus oxychloride (19.2 g, 125 mmol, 11.6 mL, 66.3 *eq*) was stirred at 110°C for 1 hour. The reaction solution was cooled to 10-20°C, and then dropped into a saturated sodium bicarbonate aqueous solution (200 mL), and extracted with ethyl acetate (150 mL) for three times. The combined organic matter was washed with saturated salt solution (30.0 mL), dried with anhydrous sodium sulfate, and filtered; and a crude product was obtained through decompression and concentration. Column chromatography was conducted on the crude product to obtain a compound 5h.

MS-ESI calculation value [M+H]⁺ 283, 284, 285; actual measurement values 283, 284, 285.

¹HNMR (400 MHz, CDCl₃) δ:8.88 (d, *J*=4.8 Hz, 1 H), 8.17 (d, *J*=9.2 Hz, 1 H), 7.50-7.44 (m, 2 H), 3.99-3.94 (m, 4 H), 3.31-3.26 (m, 4 H).

### Step VI

Pd(PPh₃)₂Cl₂ (41.3 mg, 58.9 µmol, 0.05 *eq)* and potassium acetate (347 mg, 3.53 mmol, 3 *eq*) were added into a dioxane (20 mL) solution of the compound g (409 mg, 1.18 mmol, 1 *eq*) and double-pinacol alcohol boric acid ester (448 mg, 1.77 mmol, 1.5 *eq).* The obtained reaction liquid was stirred at 130°C for 4 h. After cooling to 10-20°C, the compound 5h (200 mg, 706 µmol, 0.6 *eq*), sodium carbonate (250 mg, 2.35 mmol, 2 *eq*), Pd(dppf)Cl₂ (68.9 mg, 94.2 µmol, 0.08 *eq)* and H₂O (4.00 mL) were added. The reaction solution was replaced with nitrogen for three times and then reacted at 90°C for 2 hours. Decompression and concentration were performed to remove the solvent, water was used for diluting residue, and then ethyl acetate (150 mL) was used for extraction for three times. The combined organic matter was washed with saturated salt solution (20.0 mL), dried with anhydrous sodium sulfate, and filtered; and a crude product was obtained through concentration. After being purified by the HPLC, the crude product was then subjected to SFC (chromatographic column: Chiralcel OJ-3 150×4.6mm I.D., 3 µm; mobile phase: A: carbon dioxide B: ethanol (0.05% diethylamine); gradient: rising from 5% to 40% in 5 min, maintaining at 40% for 2.5 min, and then maintaining at 5% for 2.5 min; flow rate: 2.5 mL/min; column temperature: 35°C. Elution time: 10 min) for preparing and purification to obtain compounds 5 (retention time: 5.118 m) and 6 (retention time: 5.569 min).

### Compound 5 (retention time: 5.118 min)

MS-ESI calculation values [M+H]⁺ 515, 516, 517; actual measurement values 515, 516, 517.

¹H NMR (400 MHz, CDCl₃) δ: 9.02 (br s, 1 H), 7.55 (br d, *J*=7.6 Hz, 1 H), 7.47-7.29 (m, 4 H), 7.21 (br s, 1 H), 6.99 (d, *J*=9.2 Hz, 1 H), 6.42 (br s, 1 H), 5.12 (br s, 1 H), 4.13 (s, 3 H), 3.96 (br s, 4 H), 3.25 (br s, 4 H).

### Compound 6 (retention time: 5.569 min)

MS-ESI calculation value [M+H]⁺ 515, 516, 51; actual measurement value 515, 516, 51.

¹H NMR (400 MHz, CDCl₃) δ: 8.96 (br d, *J*=4.4 Hz, 1 H), 7.47 (d, *J*=7.6 Hz, 1 H), 7.37-7.21 (m, 4 H), 7.18-7.08 (m, 1 H), 6.92 (d, *J*=9.2 Hz, 1 H), 6.35 (s, 1 H), 4.97 (br s, 1 H), 4.05 (s, 3 H), 3.91-3.86 (m, 4 H), 3.18 (br s, 4 H).

### Embodiments 7 & 8

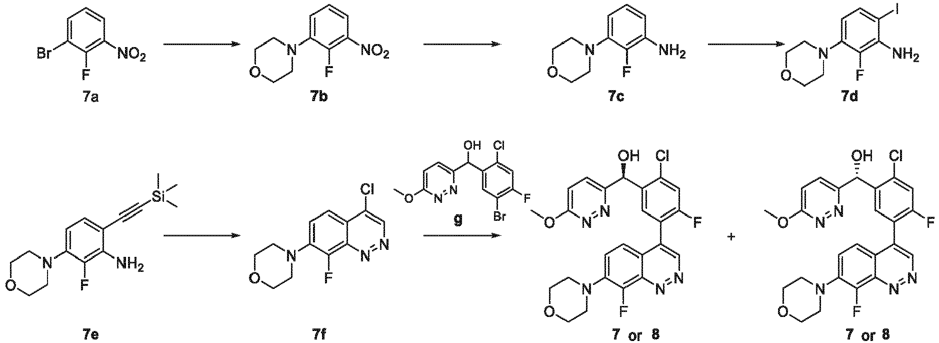

### Step I

A compound 7a (7.00 g, 31.8 mmol, 1 eq), morpholine (5.54 g, 63.6 mmol, 5.60 mL, 2 eq), cesium carbonate (20.7 g, 63.6 mmol, 2 eq), and tri(dibenzylidene acetone)dipalladium (1.46 g, 1.59 mmol, 0.05 eq) were added to anhydrous dioxane; after mixture, they were heated to 85°C and stirred for 3 h under the protection of nitrogen; after the reaction was completed, decompression and concentration were conducted. Water was added (100 mL) for dilution; ethyl acetate (100 mL^{∗}3) was used for extraction; the organic matter was washed with the saturated salt solution (30.0 mL) and dried with anhydrous sodium sulfate; decompression and concentration were conducted, and column chromatography (petroleum ether/ethyl acetate 10:1) purification was conducted to obtain a compound 7b.

MS-ESI calculation value [M+H]⁺ 227; actual measurement value 227.

¹H NMR (400 MHz, CDCl₃) δ:7.58-7.56 (m, 1H), 7.19-7.16 (m, 2H), 3.91-3.851 (m, 4H), 3.16-3.10 (m, 4H).

### Step II

The compound 7b (2.5 g, 11.1 mmol, 1 eq) was added into ethanol (25.0 mL) and water (5.00 mL). Iron powder (6.17 g, 111 mmol, 10 eq) and ammonium chloride (8.87 g, 166 mmol, 5.80 mL, 15 eq) were further added; and the mixture was stirred at 85°C for 1 hour. After the reaction was completed, decompression and concentration were performed; water (50.0 mL) was added for dilution; ethyl acetate (50.0 mL^{∗}3) extraction was performed; the organic matter was washed with saturated salt solution (30.0 mL) and dried with anhydrous sodium sulfate; decompression and concentration were performed; and column chromatography purification was conducted to obtain a compound 7c.

MS-ESI calculation value [M+H]⁺ 197; actual measurement value 197.

¹H NMR (400 MHz, DMSO-*d*₆) δ:6.78-6.718 (m, 1H), 6.42-6.40 (m, 1H), 6.19-6.16 (m, 1H), 4.97 (s, 2H), 3.78-3.66 (m, 4H), 2.96-2.87 (m, 4H).

### Step III

The compound 7c (420 mg, 2.14 mmol, 1 eq) was dissolved into anhydrous tetrahydrofuran (15.0 mL), and *N*-iodosuccinimide (578 mg, 2.57 mmol, 1.2 eq) was further added. The mixture was stirred at 20°C for 2 hours. After the reaction was completed, filtering was performed and the obtained filtrate column was subjected to column chromatography purification to obtain a compound 7c.

MS-ESI calculation value [M+H]⁺ 323; actual measurement value 323.

### Step IV

The compound 7d (432 mg, 1.34 mmol, 1 eq), trimethyl silicon acetylene (527 mg, 5.36 mmol, 743 µL, 4 eq), cuprous iodide (25.5 mg, 134 µmol, 0.1 eq), and dichloride (triphenylphosphine) palladium (II) (47.1 mg, 67.1 µmol, 0.05 eq) were added into triethylamine (5.00 mL); the mixture was stirred at 50°C for 5 h under the protection of nitrogen. After completing the reaction, decompression and concentration were performed; and column chromatography purification was performed to obtain a compound 7e.

MS-ESI calculation value [M+H]⁺ 293; actual measurement value 293.

### Step V

The compound 7e (350 mg, 1.20 mmol, 1 eq) was dissolved into diluted hydrochloric acid (3.00 mL); then the aqueous solution (1.00 mL) of sodium nitrite (124 mg, 1.80 mmol, 1.5 eq) was slowly dropped at 0°C for stirring for 30 min at this temperature, and the temperature was risen to 20°C for reaction for 2 hours. After completing the reaction, it was poured into saturated sodium bicarbonate solution (200 mL); ethyl acetate (50 mL^{∗}3) extraction was performed; the organic matter was washed with salt solution (20.0 mL), dried with anhydrous sodium sulfate; decompression and concentration were performed; and column chromatography purification was conducted to obtain a compound 7f.

MS-ESI calculation value [M+H]⁺ 268, 270; actual measurement value 268, 270.

### Step VI

The compound g (100 mg, 288 µmol, 1 *eq)* and double-pinacol alcohol boric acid ester (110 mg, 432 µmol, 1.5 *eq)* were added into anhydrous dioxane (10.0 mL), and then potassium acetate (84.7 mg, 863 µmol, 3 *eq)* and dichloride(triphenylphosphine)palladium (II) (10.1 mg, 14.4 µmol, 0.05 *eq*) were added to be stirred at 130° for 2 h under the protection of nitrogen; the compound 7f (69.3 mg, 259 µmol, 0.9 *eq*), 1,1-bis(diphenyl phosphorus) ferrocene palladium chloride (16.8 mg, 23.0 µmol, 0.08 *eq*), sodium carbonate (61.0 mg, 575 µmol, 2 *eq*), and water (2.00 mL) were then added to be reacted at 90° for 2 h under the protection of nitrogen; after the reaction was completed, most of dioxane was removed by decompression and concentration; after water (40.0 mL) was added for dilution, ethyl acetate extraction was conducted (50 mL^{∗}3); the combined organic matter was washed with saturated salt solution (20.0 mL), dried with anhydrous sodium sulfate, and filtered; and decompression and concentration were conducted. After HPLC purification, and upon chiral separation (chromatographic column: Chiralpak AD-3 50^{∗}4.6mm I.D., 3 µm); mobile phase [40% ethanol carbon dioxide solution (containing 0.05% diethylamine)]; flow rate: 4 mL/min; column temperature: 40°C; elution time: 3 min), and a compound 7 (retention time: 0.586 min) and a compound 8 (retention time: 0.830 min) were obtained through separation.

### Compound 7 (retention time: 0.586 min)

MS-ESI calculation values [M+H]⁺ 500, 502; actual measurement values 500, 502.

¹H NMR (400 MHz, DMSO-*d*₆) δ:9.33 (s, 1H), 7.90 (d, *J*=7.8 Hz, 1H), 7.80 (t, *J*=8.8 Hz, 1H), 7.74-7.72 (m, 2H), 7.55-7.50 (m, 1H), 7.21 (d, *J*=9.2 Hz, 1H), 6.65 (d, *J*=4.8 Hz, 1H), 6.23 (d, *J*=4.8 Hz, 1H), 4.00 (s, 3H), 3.85-3.78 (m, 4H), 3.39-3.36 (m, 4H).

### Compound 8 (retention time: 0.830 min)

MS-ESI calculation values [M+H]⁺ 500, 502; actual measurement values 500, 502.

¹H NMR (400 MHz, DMSO-*d*₆) δ:9.33 (s, 1H), 7.90 (d, *J*=8.0 Hz, 1H), 7.81 (t, *J*=8.8 Hz, 1H), 7.73-7.71 (m,2H), 7.55-7.50 (m, 1H), 7.21 (d, *J*=9.2 Hz, 1H), 6.64 (d, *J*=4.8 Hz, 1H), 6.23 (d, *J*=4.8 Hz, 1H), 4.00 (s, 3H), 3.87-3.73 (m, 4H), 3.39-3.32 (m,Hz, 4H).

### Embodiments 9 & 10

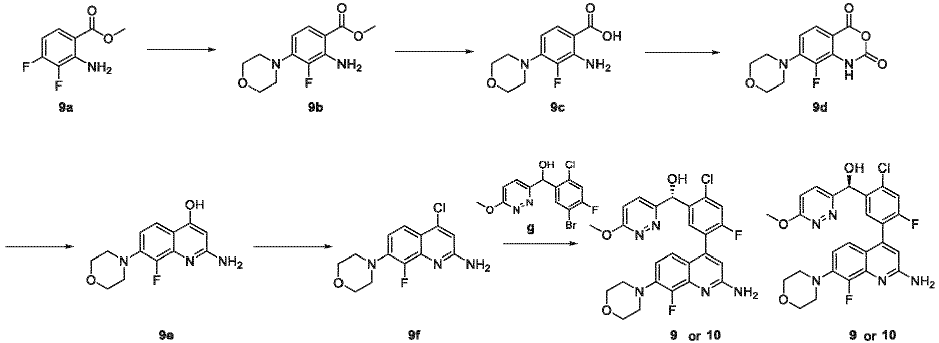

### Step I

A compound 9a (2.00 g, 10.7 mmol, 1 eq) and morpholine (6.60 g, 75.8 mmol, 6.67 mL, 7.09 eq) were mixed and then heated to 100°C, and stirred for 12 h; after the reaction was completed, decompression and concentration were conducted. Column chromatography purification was conducted to obtain a compound 9b.

MS-ESI calculation value [M+H]⁺ 255; actual measurement value 255.

¹H NMR (400 MHz, DMSO-*d*₆) δ:7.47 (dd, *J*=1.6, 8.8 Hz, 1H), 6.40 (s, 2H), 6.26 (t, *J*=8.8 Hz, 1H), 3.77 (s, 3H), 3.74-3.64 (m, 4H), 3.14-3.06 (m, 4H).

### Step II

The compound 9b (730 mg, 2.87 mmol, 1 eq) was added into tetrahydrofuran (3.00 mL) and water (2.00 mL). An aqueous solution (1.00 mL) of sodium hydroxide (230 mg, 5.74 mmol, 2 eq) was further added; the mixture was stirred at 40°C for 12 hours. After the reaction was completed, acetic acid was dropped to make a reaction solution to neutral, and a compound 9c was obtained through decompression and concentration.

MS-ESI calculation value [M+H]⁺ 241; actual measurement value 241.

### Step III

The compound 9c (1.10 g, 4.58 mmol, 1 eq) was dissolved into tetrahydrofuran (10.0 mL), and triphosgene (2.11 g, 35.8 mmol, 2.05mL, 5 eq) was further added into the reaction. The mixture was stirred at 80°C for 40 min. After the reaction was completed, the mixture was slowly poured into water (50.0 mL) and filtered to obtain a solid for decompression and concentration, and then petroleum ether (20.0 mL) was used for pulping to obtain a compound 9d.

MS-ESI calculation value [M+H]⁺ 267; actual measurement value 267.

¹H NMR (400 MHz, DMSO-*d*₆) δ:11.73 (s, 1H), 7.62 (d, *J*=8.4 Hz, 1H), 6.88 (t, *J*=8.4 Hz, 1H), 3.81-3.69 (m, 4H), 3.30-3.17 (m, 4H).

### Step IV

The compound 9d (720 mg, 2.70 mmol, 1 eq) was added to *N*,*N*-dimethylformamide (2 mL), and then *N*,*N*-dimethylformamide solution (2 mL) of malononitrile (312 mg, 3.25 mmol, 1.2 eq) and triethylamine (328 mg, 3.25 mmol, 452 µL, 1.2 eq) was dropped; the mixture was stirred at 60°C for 0.4 hours and then poured into cold 0.2 mole of dilute hydrochloric acid (14.9 mL) and filtered. After decompression and concentration, the obtained filter cake was added to 8 mole of potassium hydroxide solution (15.0 mL) and stirred at 120°C for 40 hours. After the reaction was completed, neutralized to neutral with 12 moles of hydrochloric acid, the solid was obtained by filtering and compound 9e was obtained after drying.

MS-ESI calculation value [M+H]⁺ 264; actual measurement value 264.

### Step V

The compound 9c (650 mg, 2.47 mmol, 1 eq) was dissolved into phosphorus oxychloride (9.22 g, 60.1 mmol, 5.59 mL, 24.4 eq), and the mixture was reacted at 125°C for 12 hours. Then phosphorus oxychloride was removed by decompression and concentration; the obtained solid mixture was added into water (10.0 mL); the mixture reacts at 125°C for 4 hours. After the reaction was completed, a mole of the sodium hydroxide solution was dropped to neutralize to neutral; filter; the obtained solid was decompressed and concentrated to obtain a compound 9f.

MS-ESI calculation values [M+H]⁺ 282, 284; actual measurement values 282, 284.

### Step VI

The compound g (200 mg, 575 µmol, 1 eq) and double-pinacol alcohol boric acid ester (219 mg, 863 µmol, 1.5 eq) were added into anhydrous dioxane (10.0 mL), and then potassium acetate (169 mg, 1.73 mmol, 3 eq) and dichloride(triphenylphosphine)palladium (II) (20.2 mg, 28.8 µmol, 0.05 eq) were added to be stirred at 130° for 2 h under the protection of nitrogen; the compound 9f (250 mg, 887 µmol, 1.54 eq), 1,1-bis(diphenyl phosphorus) ferrocene palladium chloride (33.7 mg, 46.0 µmol, 0.08 eq), sodium carbonate (244 mg, 2.30 mmol, 4 eq), and water (2.00 mL) were then added to be stirred at 90° for 2 h under the protection of nitrogen; after the reaction was completed, most of dioxane was removed by decompression and concentration; after water (40.0 mL) was added for dilution, ethyl acetate extraction (50.0 mL^{∗}3) was conducted; the combined organic matter was washed with saturated salt solution (20.0 mL), dried with anhydrous sodium sulfate, and filtered; and decompression and concentration were conducted. After HPLC preparation and purification and upon chiral separation (chromatographic column: Chiralcel OJ-H 150^{∗}4.6mm I.D., 5 µm); mobile phase [A phase: carbon dioxide, B phase: methanol (containing 0.05% diethylamine); gradient: maintaining B phase at 5% for 0.5 min, B phase rising from 5% to 40% in 3.5 min, maintaining at 40% for 2.5 min, and then maintaining B phase at 5% for 1.5 min]; flow rate: 3 mL/min; column temperature: 40°C; elution time: 8 min), a compound 9 (retention time: 4.607 min) and a compounds 10 (retention time: 5.167 min) were obtained through separation.

### Compound 9 (retention time: 4.607 min)

MS-ESI calculation values [M+H]⁺ 514, 516; actual measurement values 514, 516.

¹H NMR (400 MHz, DMSO-*d*₆) δ:7.69 (br d, *J*=8.8 Hz, 2H), 7.62 (br d, *J*=8.8 Hz, 1H), 7.21 (br d, J=8.8 Hz, 1H), 7.06-6.87 (m, 2H), 6.78 (br s, 2H),6.69-6.55 (m, 2H), 6.21 (br s, 1H), 4.00 (s, 3H), 3.77 (br s, 4H), 3.11 (br s, 4H).

### Compound 10 (retention time: 5.167 min)

MS-ESI calculation values [M+H]⁺ 514, 516; actual measurement values 514, 516.

¹H NMR (400 MHz, DMSO-*d*₆) δ:7.70 (dd, *J*=5.2, 8.4 Hz, 2H), 7.62 (d, *J*=9.2 Hz, 1H), 7.21 (d, J=9.2 Hz, 1H), 7.03-6.87 (m, 2H), 6.78 (br s, 2H), 6.67-6.54 (m, 2H), 6.20 (s, 1H), 4.00 (s, 3H), 3.82-3.73 (m, 4H), 3.17-3.00 (m, 4H).

### Embodiments 11 & 12

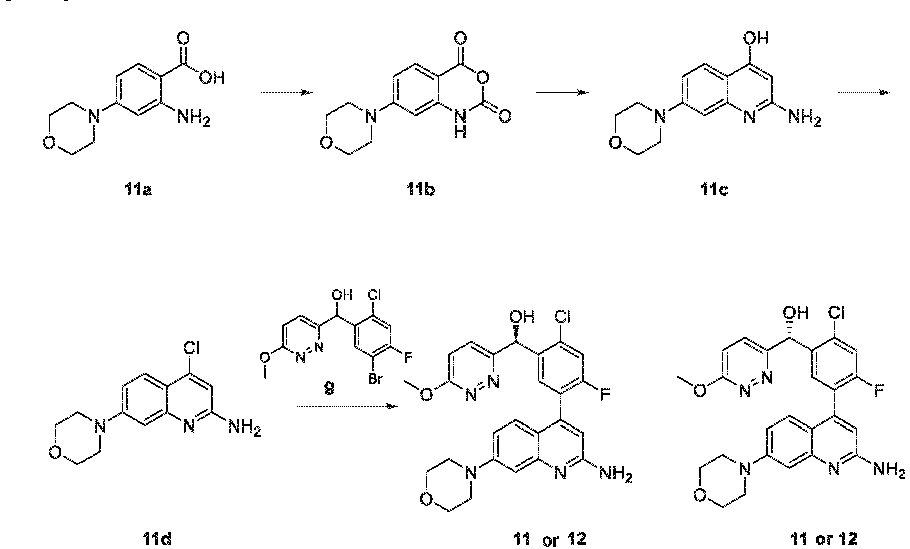

### Step I

A compound 11a (500 mg, 2.25 mmol, 1 eq) was dissolved into tetrahydrofuran (10.0 mL), and triphosgene (1.45 g, 4.89 mmol, 2.17 eq) was further added into the reaction. The mixture was stirred at 80°C for 40 min. After the reaction was completed, the mixture was slowly poured into water (50.0 mL) and filtered to obtain a solid for decompression and concentration, and then a compound 11b was obtained.

MS-ESI calculation value [M+H]⁺ 249; actual measurement value 249.

### Step II

The compound 11b (496 mg, 2.00 mmol, 1 eq) was added to *N*,*N*-dimethylformamide (2.00 mL), and then *N*,*N*-dimethylformamide solution (1 mL) of malononitrile (230 mg, 2.40 mmol, 1.2 eq) and triethylamine (243 mg, 2.40 mmol, 334 µL, 1.2 eq) was dropped; the mixture was stirred at 60°C for 0.6 hours and then poured into cold 0.2 mole of dilute hydrochloric acid (11.0 mL) and filtered. After decompression and concentration, the obtained filter cake was added to 8 mole of potassium hydroxide solution (11.1 mL) and stirred at 120°C for 40 hours. After the reaction was completed, neutralized to neutral with 12 moles of hydrochloric acid, the solid was obtained by filtering and compound 11c was obtained after drying.

MS-ESI calculation value [M+H]⁺ 246; actual measurement value 246.

### Step III

The compound 11c (500 mg, 2.04 mmol, 1 eq) was dissolved into phosphorus oxychloride (4.95 g, 32.3 mmol, 3 mL, 15.8 eq), and the mixture was reacted at 125°C for 12 hours. Then phosphorus oxychloride was removed by decompression and concentration; the obtained solid mixture was added into water (10.0 mL); the mixture reacts at 125°C for 4 hours. After the reaction was completed, a mole of the sodium hydroxide solution was dropped to neutralize to neutral; filtering was performed; and the obtained solid was decompressed and concentrated to obtain a compound 11d.

MS-ESI calculation values [M+H]⁺ 264, 266; actual measurement values 264, 266.

### Step IV

The compound g (200 mg, 575 µmol, 1 eq) and double-pinacol alcohol boric acid ester (219 mg, 863 µmol, 1.5 eq) were added into anhydrous dioxane (10.0 mL), and then potassium acetate (169 mg, 1.73 mmol, 3 eq) and dichloride(triphenylphosphine)palladium (II) (20.2 mg, 28.8 µmol, 0.05 eq) were added to be stirred at 130° for 2 h under the protection of nitrogen; the compound 11d (273 mg, 1.04 mmol, 1.8 eq), 1,1-bis(diphenyl phosphorus) ferrocene palladium chloride (33.7 mg, 46.0 µmol, 0.08 eq), sodium carbonate (122 mg, 1.15 mmol, 2 eq), and water (2.00 mL) were then added to be stirred at 90° for 2 h under the protection of nitrogen; after the reaction was completed, most of dioxane was removed by decompression and concentration; after water (40.0 mL) was added for dilution, ethyl acetate extraction (50.0 mL^{∗}3) was conducted; the combined organic matter was washed with saturated salt solution (20.0 mL), dried with anhydrous sodium sulfate, and filtered; and decompression and concentration were conducted. After HPLC preparation and purification and upon chiral separation (chromatographic column: Chiralpak AD-3 50^{∗}4.6mm I.D., 3 µm); mobile phase [40% isopropyl alcohol carbon dioxide solution (containing 0.05% diethylamine]; flow rate: 4 mL/min; column temperature: 40°C; elution time: 3 min), a compound 11 (retention time: 0.793 min) and a compound 12 (retention time: 1.203 min) were obtained through separation.

### Compound 11 (retention time: 0.793 min)

MS-ESI calculation values [M+H]⁺ 496, 498; actual measurement values 496, 498.

¹H NMR (400 MHz, DMSO-*d*₆) δ:7.69 (d, *J*=9.2 Hz, 2H), 7.61 (d, *J*=9.6 Hz, 1H), 7.21 (d, *J*=9.2 Hz, 1H), 7.10 (s, 1H), 6.96 (s, 1H), 6.86 (s, 1H), 6.59 (d, *J*=5.2 Hz, 1H), 6.50 (s, 1H), 6.36 (br s, 2H), 6.20 (d, *J*=5.2 Hz, 1H), 4.00 (s, 3H), 3.76 (br s, 4H), 3.21 (br s, 4H).

### Compound 12 (retention time: 1.203 min)

MS-ESI calculation values [M+H]⁺ 496, 498; actual measurement values 496, 498.

¹H NMR (400 MHz, DMSO-*d*₆) δ:7.69 (br d, *J*=9.2 Hz, 2H), 7.60 (d, *J*=9.2 Hz, 1H), 7.21 (d, J=9.2 Hz, 1H), 7.14-7.07 (m, 1H), 6.97-6.92 (m, 1H), 6.85 (s, 1H), 6.59 (d, *J*=5.2 Hz, 1H), 6.49 (s, 1H), 6.36 (br s, 2H), 6.20 (d, *J*=5.2 Hz, 1H), 4.00 (s, 3H), 3.76 (br s, 4H), 3.20 (br s, 4H).

### Embodiments 13 & 14

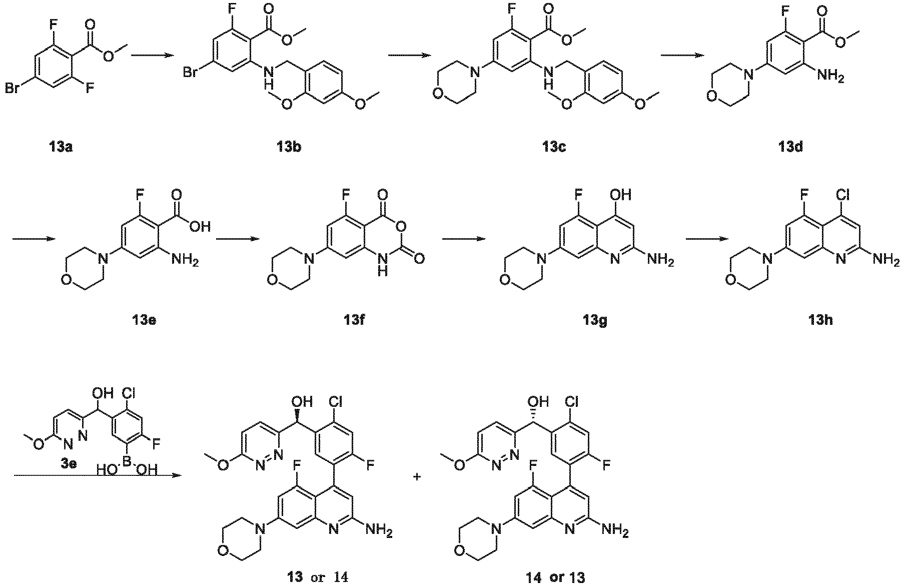

### Step I

A compound 13a (10.0 g, 39.8 mmol, 1 *eq.)* was dissolved into *N,N-*dimethylformamide (30.0 mL); then 2,4-dimethoxybenzylamine (6.66 g, 39.8 mmol, 6.00 mL, 1 *eq.)* and cesium carbonate (26.0 g, 79.7 mmol, 2 *eq.)* were added in sequence. After mixture, it was heated to 60°C, stirred and reacted for 2 hours. After the reaction was completed, water was added for dilution; ethyl acetate extraction was performed; the combined organic matter was washed with saturated salt solution and dried with anhydrous sodium sulfate. Decompression and concentration were performed; a petroleum ether and ethyl acetate mixed solution (20:1) (100 mL) was subjected to pulping to obtain a compound 13b.

¹H NMR (400 MHz, DMSO-*d*₆) δ: 7.85 (br t, *J*=5.2 Hz, 1H), 7.16 (d, *J*=8.4 Hz, 1H), 6.75 (s, 1H), 6.65 (dd, *J*=1.6, 11.2 Hz, 1H), 6.59 (d, *J*=2.0 Hz, 1H), 6.50 (dd, *J*=2.8, 8.8 Hz, 1H), 4.28 (d, *J*=5.6 Hz, 2H), 3.83 (s, 3H), 3.81 (s, 3H), 3.75 (s, 3H).

### Step II

The compound 13b (6.8 g, 17.1 mmol, 1 *eq),* morpholine (7.44 g, 85.4 mmol, 7.51 mL, 5 *eq.),* cesium carbonate (11.1 g, 34.1 mmol, 2 eq.), tri(dibenzylacetone)dipalladium (469 mg, 512 µmol, 0.03 *eq.),* and 4, 5-bis(diphenyl phosphorus)-9,9-dimethyloxanthracene (593 mg, 1.02 mmol, 0.06 *eq.)* were added into methylbenzene (40.0 mL); the mixture was heated to 110°C and stirred for 4 hours under the protection of nitrogen. After the reaction was completed, decompression and concentration were conducted. Rapid silica gel chromatography (ISCO^{®};80 g SepaFlash^{®} rapid silica gel column, eluent 0-30% ethyl acetate/petroleum ether@ 30 mL/min) purification was performed to obtain a compound 13c.

### Step III

The compound 13c (6 g, 14.8 mmol, 1 *eq.)* was dissolved in a mixture of methanol (100 mL) and tetrahydrofuran (100 mL), then wet palladium carbon (1 g, 10% purity) was added in a nitrogen atmosphere. After hydrogen replacement for 3 times, the mixture was stirred at 50°C at a hydrogen pressure of 50 Psi for 12 hours. Then filter; solid obtained after decompression and concentration of the filtrate was dissolved with dichloromethane (150 mL), and then trifluoroacetic acid (23.10 g, 202.59 mmol, 15 mL, 13.66 *eq.)* was added; the mixture was stirred at 25°C for 2 hours. After the reaction was completed, decompression and concentration were conducted. Saturated sodium carbonate solution was used for dilution; ethyl acetate extraction was performed; the combined organic matter was washed with saturated salt solution and dried with anhydrous sodium sulfate; decompression and concentration were conducted. Rapid silica gel chromatography (ISCO^{®};40 g SepaFlash^{®} rapid silica gel column, eluent 0-35% ethyl acetate/petroleum ether@ 30 mL/min) purification was performed to obtain a compound 13d.

¹H NMR (400 MHz, DMSO-*d*₆) δ: 6.66 (s, 2H), 6.04 (dd, *J*=2.0, 16.0 Hz, 1H), 5.98 (d, *J*=2.0 Hz, 1H), 3.72 (s, 3H), 3.64-3.70 (m, 4H), 3.09-3.20 (m, 4H).

### Step IV

The compound 13d (3.00 g, 11.80 mmol, 1 *eq.)* was added into a mixture solution of methanol (10.0 mL) and water (10.0 mL). Sodium hydroxide (2.36 g, 59.0 mmol, 5 eq) was further added; the mixture was stirred at 80°C for 4 hours. After the reaction was completed, most of methanol was removed through decompression and concentration, and the remaining mixture liquid was adjusted with 1 mole of hydrochloric acid at PH=5, and filtering was performed. Upon decompression and concentration of the filter cake, a compound 13e was obtained.

¹H NMR (400 MHz, DMSO-*d*₆) δ: 5.97-6.06 (m, 1H), 5.95 (d, *J*=2.4 Hz, 1H), 3.60-3.73 (m, 4H), 3.02-3.18 (m, 4H).

### Step V

The compound 13e (1 g, 4.16 mmol, 1 eq) was dissolved into tetrahydrofuran (25.0 mL), and triphosgene (2.55 g, 8.59 mmol, 2.06 eq) was further added into the reaction. The mixture was stirred at 80°C for 2 hours. After the reaction was completed, decompression and concentration were conducted to remove most of tetrahydrofuran; the remaining mixture was slowly poured into water; filter; the filter cake was decompressed and concentrated to obtain a compound 13f.

¹H NMR (400 MHz, DMSO-*d*₆) δ: 11.54 (s, 1H), 6.70 (dd, *J*=2.4, 15.2 Hz, 1H), 6.21 (d, *J*=1.6 Hz, 1H), 3.65-3.75 (m, 4H), 3.25-3.35 (m, 4H).

### Step VI

The compound 13f(430 mg, 1.62 mmol, 1 *eq.)* was added to *N*,*N*-dimethylformamide (3 mL), and then *N*,*N*-dimethylformamide solution (1 mL) of malononitrile (186 mg, 1.94 mmol, 1.2 *eq.)* and triethylamine (196 mg, 1.94 mmol, 269 µL, 1.2 *eq.)* was dropped; the mixture was stirred at 60°C for 0.6 hours and then poured into cold 0.2 mole of dilute hydrochloric acid (8.80 mL) and filtered. After decompression and concentration, the obtained filter cake was added to 8 mole of potassium hydroxide solution (20.0 mL) and stirred at 120°C for 12 hours. After the reaction was completed, the filter cake was neutralized to neutral with 6 moles of hydrochloric acid and filtered, and a compound 13g was obtained by decompressing and concentrating the filter cake.

MS-ESI calculation value [M+H]⁺ 264; actual measurement value 264.

### Step VII

The compound 13g (320 mg, 1.22 mmol, 1 *eq.)* was dissolved into phosphorus oxychloride (11.2 g, 73.2 mmol, 6.81 mL, 60.26 *eq.),* and the mixture was reacted at 125°C for 4 hours. Then phosphorus oxychloride was removed by decompression and concentration; the obtained solid mixture was added into water (20 mL); and the mixture reacts at 80°C for 0.5 hour. After the reaction was completed, saturated sodium bicarbonate aqueous solution was used for dilution; ethyl acetate extraction was performed; the combined organic matter was washed with saturated salt solution and dried with anhydrous sodium sulfate; and decompression and concentration were conducted to obtain a compound 13h.

MS-ESI calculation values [M+H]⁺ 282, 284; actual measurement values 282, 284.

### Step VIII

The compound 13h (50 mg, 177 µmol, 1 eq.), compound 3e (49.92 mg, 160 µmol, 0.9 eq.), 1,1-bis(diphenyl phosphorus) ferrocene palladium chloride (6.49 mg, 8.87 µmol, 0.05 eq.), and sodium carbonate (37.6 mg, 355 µmol, 2 eq.) were added into a mixture solution of dioxane (2.5 mL) and water (0.5 mL) to be stirred at 96° for 3 h under the protection of nitrogen; after the reaction was completed, filter; decompression and concentration were conducted. HPLC (separation column: Xtimate C18 150×25mm×5µm; mobile phase: [water (0.225% formic acid)-acetonitrile]; B%: 30%-60%, 7 min) preparation and purification were performed on supercutical fluid (chromatographic column: Chiralcel OD-3 100x4.6mm I.D., 3 µm); mobile phase: [40% ethanol carbon dioxide solution (containing 0.05% diethylamine)]; flow rate: 2.8 mL/min; column temperature: 40°C; elution time: 8 min), and compounds 13 (retention time: 5.171 min) and 14 (retention time: 5.765 min) were obtained.

### Compound 13 (retention time: 5.171 min)

MS-ESI calculation values [M+H]⁺ 514, 516; actual measurement values 514, 516.

¹H NMR (400MHz, DMSO-*d*₆) δ: 7.60-7.75 (m, 2H), 7.48 (br d, *J*=8.4 Hz, 1H), 7.15-7.27 (m, 1H), 6.74 (s, 1H), 6.68 (s, 1H), 6.59 (br d, *J*=4.4 Hz, 2H), 6.55 (br s, 1H), 6.40 (d, *J*=6.4 Hz, 1H), 6.18 (s, 1H), 4.00 (d, *J*=6.0 Hz, 3H), 3.74 (s, 4H), 3.21 (s, 4H).

### Compound 14 (retention time: 5.765 min)

MS-ESI calculation values [M+H]⁺ 514, 516; actual measurement values 514, 516.

¹H NMR (400 MHz, DMSO-*d*₆) δ: 7.61-7.72 (m, 2H), 7.48 (br d, *J*=8.0 Hz, 1H), 7.17-7.25 (m, 1H), 6.74 (s, 1H), 6.68 (s, 1H), 6.58-6.64 (m, 2H), 6.56 (br s, 1H), 6.40 (d, *J*=6.4 Hz, 1H), 6.18 (s, 1H), 4.00 (d, *J*=6.0 Hz, 3H), 3.73 (s, 4H), 3.21 (s, 4H).

### Embodiments 15 & 16

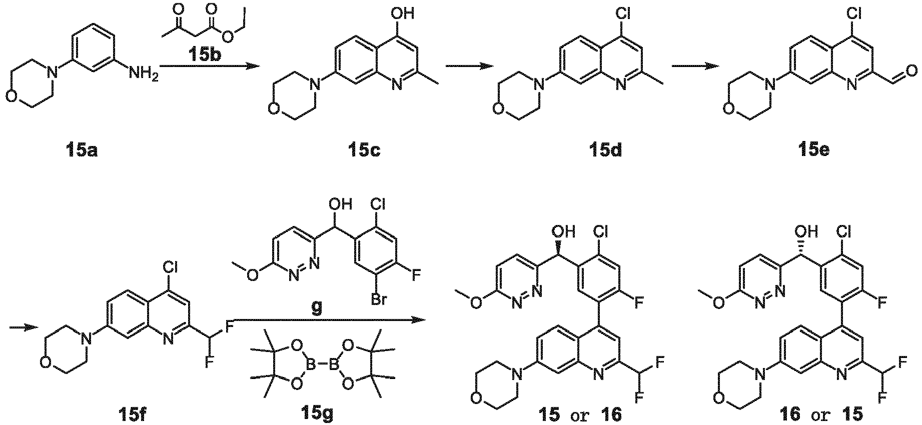

### Step I

Phosphorus pentoxide (796 mg, 5.61 mmol, 346 µL, 2 eq.) was added to a mixture system of loprazolam (2 mL) of a compound 15a (0.5 g, 2.81 mmol, 1 eq.) and a compound 15b (475 mg, 3.65 mmol, 461 µL, 1.3 eq.). The reaction system reacts at 135°C for 2 hours. After the reaction was completed, the reaction solution was slowly poured into 30 mL of ice water, then the system was adjusted using 2M of the sodium bicarbonate solution as Ph>7; filter; the filter cake was collected; and after decompression and drying, a compound 15c was obtained.

MS-ESI calculation value [M+H]⁺ 245; actual measurement value 245.

¹H NMR (400 MHz, CDCl₃) δ: 7.69-7.67 (m, 1H), 7.55-7.53 (m, 1H), 7.23 (s, 1H), 7.11-7.09 (m, 1H), 3.97-3.89 (m, 2H), 3.88-3.82 (m, 2H), 3.06-3.04 (m, 2H), 2.88-2.86 (m, 2H), 2.60 (s, 3H).

### Step II

A mixture system of the compound 15c (8.5 g, 34.8 mmol, 1 eq) and phosphorus oxychloride (8.55 g, 512 mmol, 47.6 mL, 14.7 eq) was reacted at 110°C for 1 hour. After the reaction was completed, decompression and concentration were conducted to remove most of phosphorus oxychloride. Water (300 mL) was added for dilution and saturated sodium carbonate solution was used for adjusting the system pH>7; dichloromethane extraction was performed; the organic matter was washed with saturated salt solution (200 mL), dried with anhydrous sodium sulfate, and filtered; and decompression and concentration were conducted. Rapid silica gel chromatography (ISCO^{®};80 g SepaFlash^{®} rapid silica gel column, eluent 0-100% ethyl acetate/petroleum ether@ 20 mL/min) purification was performed to obtain a compound 15d.

MS-ESI calculation values [M+H]⁺ 263, 265; actual measurement values 263, 265.

¹H NMR (400 MHz, CDCl₃) δ: 8.05-7.99 (m, 1H), 7.31-7.27 (m, 2H), 7.17 (s, 1H), 3.93-3.87 (m, 4H), 3.38-3.30 (m, 4H), 2.69-2.62 (s, 3H).

### Step III

Selenium dioxide (2.11 g, 19 mmol, 2.07 mL, 2.5 eq.) was added to xylene (50 mL) solution of the compound 15d (2 g, 7.61 mmol, 1 eq.), and the reaction system reacts for 5 hours at 100°C in nitrogen atmosphere. After the reaction was completed, filter; the filter cake was decompressed and concentrated; rapid silica gel chromatography (ISCO^{®}; 20 g rapid silica gel column, eluent 0-100%-petroleum ether/ethyl acetate, flow rate 10 mL/min) was performed to obtain a mixture15e.

¹H NMR (400 MHz, CDCl₃) δ: 10.05 (s, 1H), 8.08 (d, *J*=9.2 Hz, 1H), 7.80 (s, 1H), 7.47-7.37 (m, 2H), 3.89-3.83 (m, 4H), 3.38-3.30 (m, 4H).

### Step IV

Diethylamino sulfur trifluoride (373 mg, 2.31 mmol, 306 µL, 2 eq.) was added into a dichloromethane solution (8 mL) of the compound 15e (0.32 g, 1.16 mmol, 1 eq.). The reaction system reacts at 25°C for 2 hours. After the reaction was completed, water (10 mL) was added into the reaction liquid and dichloromethane extraction was performed; the organic matter was washed with saturated salt solution (20 mL) and dried with anhydrous sodium sulfate; and decompression and concentration were conducted. Rapid silica gel chromatography (ISCO^{®};12 g rapid silica gel column, eluent 0-100% petroleum ether/ethyl acetate, flow rate 10 mL/min) purification was performed to obtain a compound 15f.

MS-ESI calculation values [M+H]⁺ 299, 301; actual measurement values 299, 301.

### Step V

A mixture liquid of dioxane (2 mL) of a compound g (0.05 g, 144 µmol, 1 eq.), 15g (54.8 mg, 216 µmol, 1.5 eq.), potassium acetate (42.4 mg, 432 µmol, 3 eq.), and dichloride (triphenylphosphine) Palladium (II) (5.05 mg, 7.19 µmol, 0.05 eq.) was reacted at 130°C for 4 h in a nitrogen atmosphere; after cooling to room temperature, 15f (25.8 mg, 86.3 µmol, 0.6 eq.), potassium carbonate (59.7 mg, 432 µmol, 3 eq.), 1,1-bis(tert-butylphosphorous) ferrocene palladium chloride (4.69 mg, 7.19 µmol, 0.05 eq), and water (0.4 mL) were further added; the mixture reacts at 115°C for 14 h in the nitrogen atmosphere. After the reaction was completed, filtering was performed, and the filtrate was decompressed and concentrated. HPLC (separation column: Ultralimit column C18 150×25mm×5µm; mobile phase: [water (0.225% formic acid)-acetonitrile]; B%: 42%-72%, 7 min) preparation and purification were performed, supercutical fluid chromatography (SFC) (chromatographic column: Chiralpak AD-3 50^{∗}4.6mm I.D., 3 µm); mobile phase: [40% ethanol carbon dioxide solution (containing 0.05% diethylamine)]; flow rate: 4 mL/min; column temperature: 40°C; elution time: 3 min), and compounds 15 (retention time: 0.487 min) and 16 (retention time: 0.756 min) were obtained.

### Compound 15 (retention time: 0.487 min)

MS-ESI calculation values [M+H]⁺ 531, 533; actual measurement values 531, 533.

¹H NMR (400 MHz, acetonitrile-d) δ: 7.74 (d, *J*=7.6 Hz, 1H), 7.61 (d, *J*=92 Hz, 2H), 7.52-7.45 (m, 3H), 7.40 (d, *J*=2.4 Hz, 1H), 7.08 (d, *J*=9.2 Hz, 1H), 7.00-6.70 (m, 1H), 6.37 (d, *J*=5.2 Hz, 1H), 4.62 (d, *J*=4.8 Hz, 1H), 4.08 (s, 3H), 3.88-3.83 (m, 4H), 3.41-3.36 (m, 4H).

### Compound 16 (retention time: 0.756 min)

MS-ESI calculation values [M+H]⁺ 531, 533; actual measurement values 531, 533.

¹H NMR (400 MHz, acetonitrile-d) δ: 7.74 (d, *J*=8.0 Hz, 1H), 7.61 (d, *J*=9.2 Hz, 2H), 7.52-7.44 (m, 3H), 7.40 (d, *J*=2.4 Hz, 1H), 7.08 (d, *J*=9.2 Hz, 1H), 7.00-6.70 (m, 1H), 6.37 (d, *J*=4.8 Hz, 1H), 4.63 (d, *J*=5.2 Hz, 1H), 4.08 (s, 3H), 3.88-3.83 (m, 4H), 3.42-3.36 (m, 4H).

### Embodiments 17 & 18

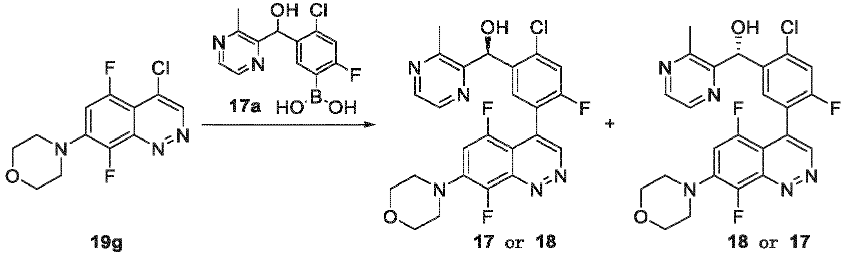

A compound 19g (80 mg, 280 µmol, 1 *eq*), compound 17a (351 mg, 1.18 mmol, 3.9 *eq.*)*,* bis(tricyclohexylphosphonyl) palladium dichloride (II) (16.5 mg, 22.4 µmol, 0.08 *eq.),* and cesium carbonate (183 mg, 560 µmol, 2 *eq.)* were added into a mixture solvent of dioxane (10 mL) and water (2 mL) to be stirred at 90° for 5 h under the protection of nitrogen; after the reaction was completed, decompression and concentration were conducted. HPLC (separation column: Xtimate C₁₈ 150×25mm,5µm; mobile phase: [water (0.225% formic acid)-acetonitrile]; B%: 42%-62%, 7 min) preparation and purification were performed, supercutical fluid chromatography (SFC) ((chromatographic column: Chiralpak AD-3 50^{∗}4.6mm I.D., 3 µm); mobile phase: [40% ethanol carbon dioxide solution (containing 0.05% diethylamine)]; flow rate: 4 mL/min; column temperature: 35°C; elution time: 2 min)), and a compound 17 (retention time: 0.538 min) and a compound 18 (retention time: 1.008 min) were obtained.

### Compound 17 (retention time: 0.538 min)

MS-ESI calculation values [M+H]⁺ 502, 504; actual measurement values 502, 504.

¹H NMR (400 MHz, DMSO-*d*₆) δ: 9.24 (br d, *J*=4.0 Hz, 1H), 8.48-8.29 (m, 2H), 7.90 (br t, *J*=7.2 Hz, 1H), 7.76-7.67 (m, 1H), 7.60 (br d, *J*=9.6 Hz, 1H), 6.47 (br s, 1H), 6.26-6.20 (m, 1H), 3.83-3.76 (m, 4H), 3.45-3.40 (m, 4H), 2.71 (br d, *J*=17.2 Hz, 3H).

### Compound 18 (retention time: 1.008 min)

MS-ESI calculation values [M+H]⁺ 502, 504; actual measurement values 502, 504.

¹H NMR (400 MHz, DMSO-*d*₆) δ: 9.25 (br d, *J*=3.6 Hz, 1H), 8.51-8.28 (m, 2H), 7.91 (br t, *J*=7.6 Hz, 1H), 7.80-7.68 (m, 1H), 7.61 (br d, *J*=9.6 Hz, 1H), 6.48 (br s, 1H), 6.24 (br s, 1H), 3.83-3.76 (m, 4H), 3.44-3.41 (m, 4H), 2.72 (br d, *J*=17.6 Hz, 3H).

### Embodiments 19 & 20

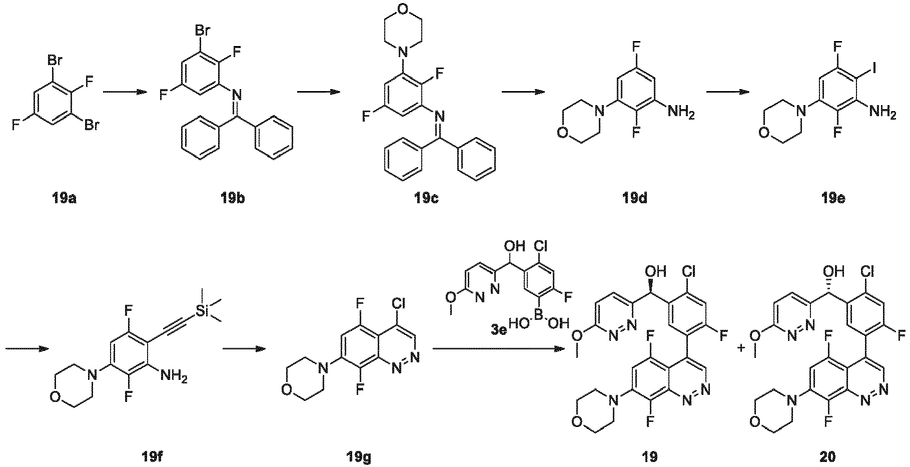

### Step I

A methylbenzene mixture solution (260 mL) of the compound 19a (26 g, 95.6 mmol, 1 *eq*)*,* benzophenone imine (17.3 g, 95.6 mmol, 16.1 mL, 1 *eq*), Pd₂(dba)₃ (2.63 g, 2.87 mmol, 0.03 *eq*)*,* BINAP (5.95 g, 9.56 mmol, 0.1 *eq*) and *t*-BuONa (13.8 g, 143 mmol, 1.5 *eq*) was subjected to nitrogen replacement for three times and then reacts in this atmosphere for 16 hours at 80°C. Decompression and concentration were performed to remove the solvent and then silica gel column purification (petroleum ether:ethyl acetate = 10:1) was conducted to obtain a compound 19b.

MS-ESI calculation values [M+H]⁺ 372, 374; actual measurement values 372, 374.

¹HNMR (400 MHz, CDCl₃) δ: 7.79-7.74 (m, 2 H), 7.56-7.50 (m, 1H), 7.47-7.41 (m, 2H), 7.34 (d, *J*=7.6 Hz , 2H), 7.18-7.13 (m, 2H), 6.86-6.84 (m, 1H), 6.48-6.45 (m, 1H).

### Step II

A 210 mL of methylbenzene solution of the compound 19b (21 g, 56.4 mmol, 1 *eq),* morpholine (9.83 g, 113 mmol, 9.93 mL, 2 *eq),* BINAP (3.51 g, 5.64 mmol, 0.1 *eq*)*, t*-BuONa (8.13 g, 84.6 mmol, 1.5 *eq*) and Pd₂(dba)₃ (1.55 g, 1.69 mmol, 0.03 *eq*) was replaced with nitrogen for three times and reacts at 120°C for 16 hours in this atmosphere. After decompression and concentration were performed to remove the solvent, 200 mL of water was used for dilution, and then 200 mL of ethyl acetate was used for extraction twice. The combined organic matter was washed with 200 mL saturated salt solution, dried with anhydrous sodium sulfate; and a crude product was obtained through decompression and concentration. Upon silica gel column purification (petroleum ether:ethyl acetate = 10:1) was conducted, a compound 19c was obtained .

MS-ESI calculation value [M+H]⁺ 379; actual measurement value 379.

### Step III

Pd/C (10%, 6.8 g) was added to a 300 mL of methanol solution of the compound 19c (21 g, 55.5 mmol, 1 *eq*) in a nitrogen atmosphere. The reaction solution was replaced with hydrogen for three times (50 Psi) and stirred at 60°C for 16 hours. The reaction solution was filtered by kieselguhr, and then was decompressed and concentrated to obtain the crude product. After the crude product was subjected to silica gel column purification (petroleum ether:ethyl acetate = 3:1), a compound 19c was obtained..

MS-ESI calculation value [M+H]⁺ 215; actual measurement value 215.

¹HNMR (400 MHz, CDCl₃) δ: 6.17-6.15 (m, 1H), 6.08-6.03 (m, 1H), 3.85-3.88 (m, 4H), 3.79 (s, 2H), 3.04-3.08 (m, 4H).

### Step IV

The compound 19d (5.5 g, 25.7 mmol, 1 *eq.*) was dissolved into anhydrous tetrahydrofuran (50 mL), and *N*-iodosuccinimide (6.07 g, 27.0 mmol, 1.05 *eq.*) was further added. The mixture was stirred at 25°C for 1 hour. After the reaction was completed, filtering was performed, decompression and concentration were conducted. Rapid silica gel chromatography (ISCO^{®}; 80 g rapid silica gel column, eluent 0-8% ethyl acetate/petroleum ether flow rate@ 60 mL/min) purification was performed to obtain a compound 19e.

MS-ESI calculation value [M+H]⁺ 341; actual measurement value 341.

¹H NMR (400 MHz, DMSO-*d*₆) δ: 6.24 (dd, *J*=6.8, 10.0 Hz, 1H), 5.32 (s, 2H), 3.75-3.66 (m, 4H), 3.01-2.90 (m, 4H).

### Step V

The compound 19e (2 g, 5.88 mmol, 1 *eq*)*,* trimethyl silicon acetylene (1.73 g, 17.6 mmol, 2.44 mL, 3 *eq.*)*,* cuprous iodide (112 mg, 588 µmol, 0.1 *eq*), and dichloride (triphenylphosphine) palladium (II) (206 mg, 294 µmol, 0.05 *eq.*) were added into triethylamine (60 mL); the mixture was stirred at 50°C for 2 h under the protection of nitrogen. After the reaction was completed, filter, decompression and concentration were conducted. Rapid silica gel chromatography (ISCO^{®}; 40 g rapid silica gel column, eluent 0-10% ethyl acetate/petroleum ether flow rate 35 mL/min) purification was performed to obtain a compound 19f.

MS-ESI calculation value [M+H]⁺ 311; actual measurement value 311.

### Step VI

The compound 19f (1.6 g, 5.15 mmol, 1 *eq.*) was dissolved into concentrated hydrochloric acid (10 mL); then the aqueous solution (2 mL) of sodium nitrite (533 mg, 7.73 mmol, 1.5 *eq.*) was dropped at 0°C for stirring for 30 min at this temperature, and the temperature was risen to 20°C for reacting for 1 hour. After the reaction was completed, it was poured into a saturated solution of sodium bicarbonate to adjust the alkali to pH=8; dichloromethane extraction was performed; the organic matter was washed with saturated salt solution and dried with anhydrous sodium sulfate; and decompression and concentration were conducted. Rapid silica gel chromatography (ISCO^{®}; 12 g rapid silica gel column, eluent 0-50% ethyl acetate/dichloromethane flow rate 30 mL/min) purification was performed to obtain a compound 19g.

MS-ESI calculation values [M+H]⁺ 286, 288; actual measurement values 286, 288.

¹H NMR (400 MHz, DMSO-*d*₆) δ: 9.43 (s, 1H), 7.80 (dd, *J*=6.8, 14.4 Hz, 1H), 3.81-3.76 (m, 4H), 3.40-3.43 (m, 4H).

### Step VII

The compound 19g (0.65 g, 2.28 mmol, 1 *eq.*), compound 3e (2.5 g, 8.00 mmol, 3.52 *eq.*)*,* 1,1-bis(diphenyl phosphorus) ferrocene palladium chloride (83.2 mg, 114 µmol, 0.05 *eq.*)*,* and sodium carbonate (723 mg, 6.83 mmol, 3 *eq.*) were added into a mixture solvent of dioxane (30 mL) and water (5 mL) and stirred at 95°C for 3 hours under the protection of nitrogen. After the reaction was completed, filter, and decompression and concentration were conducted. HPLC (separation column: Xtimate C18 150×25mm,5µm; mobile phase: [water (10 mM ammonium bicarbonate)-acetonitrile]; B%: 42%-62%, 7 min) preparation and purification were performed, supercutical fluid chromatography (SFC) (chromatographic column: Chiralpak AD-3 50^{∗}4.6mm I.D., 3 µm); mobile phase: [40% ethanol carbon dioxide solution (containing 0.05% diethylamine)]; flow rate: 4 mL/min; column temperature: 35°C; elution time: 2 min), and a compound 19 (retention time: 0.671 min) and a compound 20 (retention time: 1.128 min) were obtained.

### Compound 19 (retention time: 0.671 min)

MS-ESI calculation values [M+H]⁺ 518, 520; actual measurement values 518, 520.

¹H NMR (400 MHz, DMSO-*d*₆) δ: 9.35-9.18 (m, 1H), 7.91 (br t, *J*=7.6 Hz, 1H), 7.82-7.61 (m, 3H), 7.28-7.17 (m, 1H), 6.65 (br s, 1H), 6.23 (br s, 1H), 4.01 (br d, *J*=10.4 Hz, 3H), 3.81 (m, 4H), 3.41 (m, 4H).

### Compound 20 (retention time: 1.128 min)

MS-ESI calculation values [M+H]⁺ 518, 520; actual measurement values 518, 520.

¹H NMR (400 MHz, DMSO-*d*₆) δ: 9.35-9.18 (m, 1H), 7.91 (br t, *J*=8.4 Hz, 1H), 7.80-7.59 (m, 3H), 7.28-7.17 (m, 1H), 6.65 (br s, 1H), 6.23 (br s, 1H), 4.01 (br d, *J*=10.4 Hz, 3H), 3.81 (m, 4H), 3.41 (m, 4H).

### Embodiments 21 & 22

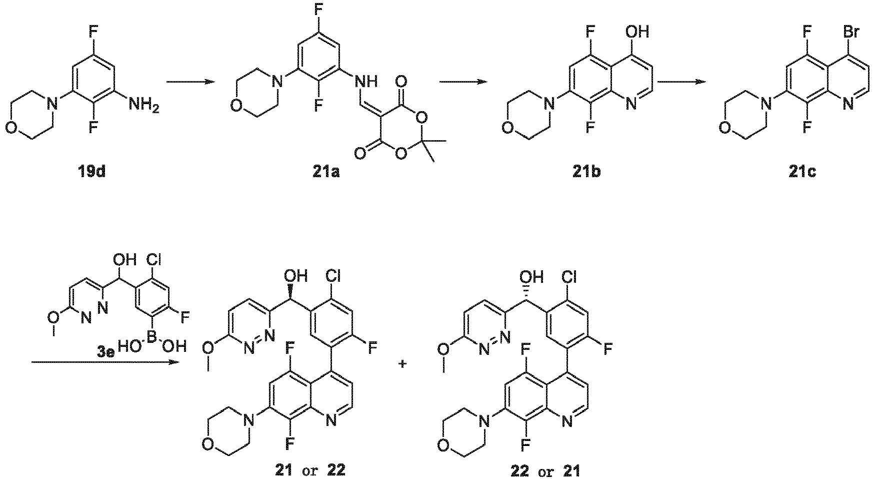

### Step I

A compound 19d (7 g, 32.7 mmol, 1 *eq.*) was dissolved into triethyl orthoformate (71.3 g, 481 mmol, 80 mL, 14.7 *eq.*)*,* and isopropyl malonate (12.5 g, 86.7 mmol, 2.65 *eq.*) was further added. The temperature was risen to 145°C for stirring for 3 hours. After the reaction was completed, it was cooled to the room temperature; filter; the filter cake was washed by isopropyl ether and decompressed and dried to obtain a compound 21a.

MS-ESI calculation value [M+H]⁺ 369; actual measurement value 369.

¹H NMR (400 MHz, DMSO-*d*₆) δ: 11.39 (br d, *J*=13.6 Hz, 1H), 8.72 (d, *J*=13.6 Hz, 1H), 7.49-7.44 (m, 1H), 6.79-6.74 (m, 1H), 3.81-3.67 (m, 4H), 3.14-3.00 (m, 4H), 1.68 (s, 6H).

### Step II

The compound 21a (8 g, 21.7 mmol, 1 *eq.*) and diphenyl ether (107 g, 629 mmol, 100 mL, 28.94 *eq.*) were stirred at 240°C for 1 hour. After the reaction was completed, it was cooled to the room temperature; isopropyl ether was used for dilution, and then filtering was performed; the filter cake was decompressed and dried to obtain a compound 21b.

MS-ESI calculation value [M+H]⁺ 267; actual measurement value 267.

¹HNMR (400 MHz, DMSO-*d*₆) δ: 11.44 (br d, *J*=4.0 Hz, 1H), 7.66 (dd, *J*=6.0, 7.2 Hz, 1H), 6.75 (dd, *J*=6.8, 14.0 Hz, 1H), 5.87 (d, *J*=8.0 Hz, 1H), 3.80-3.67 (m, 4H), 3.26-3.05 (m, 4H).

### Step III

The compound 21b (5.45 g, 20.5 mmol, 1 *eq.*) was dissolved in anhydrous *N,N-*dimethylformamide (100 mL) and then phosphorus tribromide (8.31 g, 30.7 mmol, 1.5 *eq.*) was slowly dropped; the mixture was stirred and reacted at 25°C for 0.5 hour. After the reaction was completed, saturated sodium carbonate solution (200 mL) was poured in; dichloromethane extraction was performed; the organic matter was washed with saturated salt solution and dried with anhydrous sodium sulfate; and decompression and concentration were conducted. Petroleum ether pulping was conducted to obtain a compound 21c.

MS-ESI calculation values [M+H]⁺ 329, 331; actual measurement values 329, 331.

¹H NMR (400 MHz, DMSO-*d*₆) δ: 8.63 (d, *J*=4.4 Hz, 1H), 7.80 (d, *J*=4.8 Hz, 1H), 7.44 (dd, *J*=7.2,4.8 Hz, 1H), 3.80-3.75 (m, 4H), 3.31-3.26 (m, 4H).

### Step IV

The compound 21c (1 g, 3.04 mmol, 1 *eq.*)*,* compound 3e (3.5 g, 11.2 mmol, 3.68 *eq.*)*,* 1,1-bis(diphenyl phosphorus) ferrocene palladium chloride (111 mg, 152 µmol, 0.05 *eq.*)*,* and sodium carbonate (644 mg, 6.08 mmol, 2 *eq.*) were added into a mixture solvent of dioxane (30 mL) and water (5 mL) and reacted at 95°C for 3 hours under the protection of nitrogen. After the reaction was completed, decompression and concentration were conducted. Water was added for dilution; ethyl acetate extraction was conducted; and the combined organic matter was washed with saturated salt solution and dried with anhydrous sodium sulfate. decompression and concentration; rapid silica gel chromatography (ISCO^{®}; 20 g rapid silica gel column, eluent 0-100% ethyl acetate/petroleum ether flow rate 35 mL/min) purification was performed; supercutical fluid chromatography (SFC) (chromatographic column: Chiralpak AD-3 50x4.6mm I.D., 3 µm; mobile phase: [40% ethanol carbon dioxide solution (containing 0.05% diethylamine)]; flow rate: 4 mL/min; column temperature: 35°C; elution time: 2.5 min), and a compound 21 (retention time: 0.806 min) and a compound 22 (retention time: 1.224 min) were obtained.

### Compound 21 (retention time: 0.806 min)

MS-ESI calculation values [M+H]⁺ 517, 519; actual measurement values 517, 519.

¹H NMR (400 MHz, DMSO-*d*₆) δ: 8.97 (br s, 1H), 7.79 (br s, 1H), 7.75-7.60 (m, 1H), 7.56 (br d, *J*=8.8 Hz, 1H), 7.38 (br s, 2H), 7.21 (br s, 1H), 6.60 (br s, 1H), 6.21 (br s, 1H), 4.01 (br d, *J*=6.4 Hz, 3H), 3.87-3.70 (m, 4H), 3.37-3.20 (m, 4H).

### Compound 22 (retention time: 1.224 min)

MS-ESI calculation values [M+H]⁺ 517, 519; actual measurement values 517, 519.

¹H NMR (400 MHz, DMSO-*d*₆) δ: 8.92-9.03 (m, 1H), 7.82-7.77 (m, 1H), 7.73-7.60 (m, 1H), 7.56 (d, *J*=9.2 Hz, 1H), 7.42-7.28 (m, 2H), 7.20 (dd, *J*=5.2, 9.6 Hz, 1H), 6.63-6.57 (m, 1H), 6.21 (br d, *J*=3.6 Hz, 1H), 4.01 (d, *J*=7.6 Hz, 3H), 3.83-3.73 (m, 4H), 3.33-3.22 (m, 4H).

### Embodiments 23 & 24

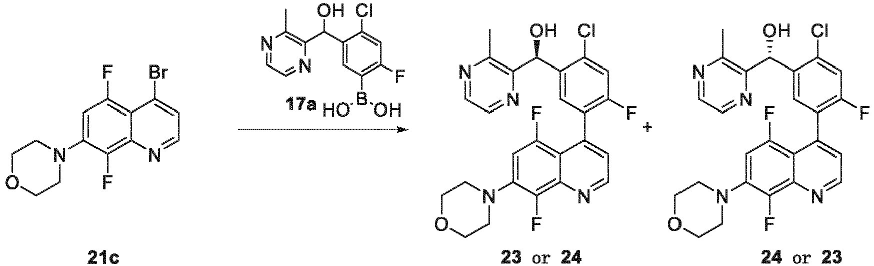

### Step I

The compound 21c (100 mg, 304 µmol, 1 *eq*.), compound 17a (351 mg, 1.18 mmol, 3.9 *eq.*)*,* 1,1-bis(diphenyl phosphorus) ferrocene palladium chloride (17.8 mg, 24.3 µmol, 0.08 *eq.*)*,* and sodium carbonate (96.6 mg, 911 µmol, 3 *eq.*) were added into a mixture solvent of dioxane (5 mL) and water (1 mL) to be reacted at 95° for 3 h under the protection of nitrogen; after the reaction was completed, decompression and concentration were conducted. HPLC (separation column: Xtimate C18 150×25mm,5µm; mobile phase: [water (0.225% formic acid)-acetonitrile]; B%: 42%-62%, 7 min) preparation and purification were performed, supercutical fluid chromatography (SFC) ((chromatographic column: Chiralpak IG-3 50x4.6mm I.D., 3 µm); mobile phase: [40% ethanol carbon dioxide solution (containing 0.05% diethylamine)]; flow rate: 4 mL/min; column temperature: 35°C; elution time: 3 min), and a compound 23 (retention time: 0.926 min) and a compound 24 (retention time: 1.512 min) were obtained.

### Compound 23 (retention time: 0.926 min)

MS-ESI calculation values [M+H]⁺ 501, 503; actual measurement values 501, 503.

¹H NMR (400 MHz, DMSO-*d*₆) δ: 8.99 (d, *J*=4.0 Hz, 1H), 8.43 (dd, *J*=2.4, 9.6 Hz, 1H), 8.35 (dd, *J*=2.4, 11.6 Hz, 1H), 7.81 (t, *J*=8.8 Hz, 1H), 7.52 (d, *J*=9.2 Hz, 1H), 7.41-7.29 (m, 2H), 6.45 (br s, 1H), 6.21 (d, *J*=4.4 Hz, 1H), 3.76-3.82 (m, 4H), 3.30-3.25 (m, 4H), 2.74-2.67 (m, 3H).

### Compound 24 (retention time: 1.512 min)

MS-ESI calculation values [M+H]⁺ 501, 503; actual measurement values 501, 503.

¹H NMR (400 MHz, DMSO-*d*₆) δ: 8.98 (d, *J*=4.0 Hz, 1H), 8.48-8.29 (m, 2H), 7.81 (t, *J*=8.8 Hz, 1H), 7.52 (br d, *J*=9.2 Hz, 1H), 7.42-7.27 (m, 2H), 6.44 (br s, 1H), 6.21 (br s, 1H), 3.83-3.73 (m, 4H), 3.33-3.23 (m, 4H), 2.75-2.67 (m, 3H).

### Embodiments 25 & 26

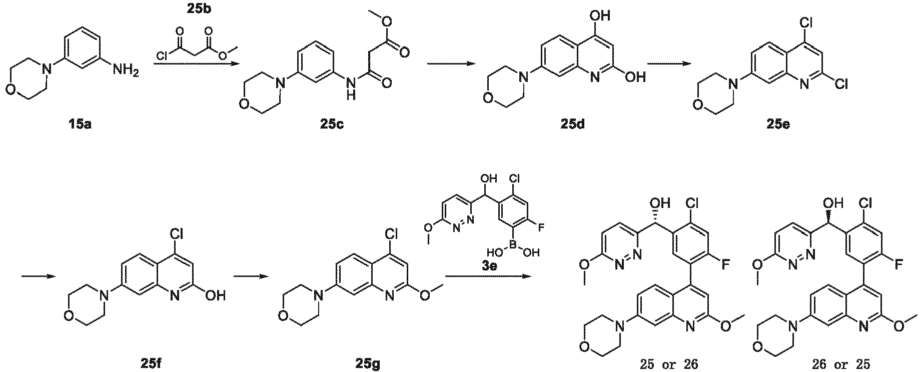

### Step I

A compound 15a (500 mg, 2.25 mmol, 1 eq) was dissolved into tetrahydrofuran (10.0 mL), and 25b (1.45 g, 4.89 mmol, 2.17 eq) was further added into the reaction. The mixture was stirred at 80°C for 40 min. After the reaction was completed, the mixture was slowly poured into water (50.0 mL) and filtered to obtain a compound 25c.

MS-ESI calculation value [M+H]⁺ 279; actual measurement value 279.

### Step II

The compound 25c (496 mg, 2.00 mmol, 1 eq) was added to *N,N*-dimethylformamide (2.00 mL), and then *N,N*-dimethylformamide solution (1.00 mL) of malononitrile (230 mg, 2.40 mmol, 1.2 eq) and triethylamine (243 mg, 2.40 mmol, 334 µL, 1.2 eq) was dropped; the mixture was stirred at 60°C for 0.6 hour and then poured into cold 0.2 mole of dilute hydrochloric acid (11.0 mL) and filtered. After decompression and concentration, the obtained filter cake was added to 8 mole of potassium hydroxide solution (11.1 mL) and stirred at 120°C for 40 hours. After the reaction was completed, the filter cake was neutralized to neutral with 12 moles of hydrochloric acid and filtered, and a compound 25d was obtained after drying.

MS-ESI calculation value [M+H]⁺ 247; actual measurement value 247.

### Step III

The compound 25d (500 mg, 2.04 mmol, 1 eq) was dissolved into phosphorus oxychloride (4.95 g, 32.3 mmol, 3 mL), and the mixture was reacted at 125°C for 12 hours. Then phosphorus oxychloride was removed by decompression and concentration; the obtained solid mixture was added into water (10.0 mL); the mixture reacts at 125°C for 4 hours. After the reaction was completed, a mole of the sodium hydroxide solution was dropped to neutralize to neutral; filter to obtain a compound 25e.

MS-ESI calculation values [M+H]⁺ 283, 284, 285; actual measurement values 283, 284, 285.

### Step IV

The compound 25e (50 mg, 0.177 mmol) was dissolved in concentrated hydrochloric acid (510 mg, 4.76 mmol) and the reaction solution reacts at 65°C. After fully reacted, the reaction solution was filtered and dried to obtain a compound 25f.

MS-ESI calculation values [M+H]⁺ 265, 267; actual measurement values 265, 267.

### Step V

The compound 25f (40 mg, 151 µmol, 1 eq) was dissolved in *N,N*-dimethylformamide (2.00 mL), and silver oxide (37.4 mg, 302 µmol, 5.00 µL, 2 eq), methane iodide (0.4 g, 2.82 mmol, 175 µL, 18.7 eq), potassium carbonate (41.8 mg, 302 µmol, 2 eq) and *N,N-*diisopropylethylamine (58.6 mg, 453 µmol, 78.9 µL, 3 eq) were further added. The mixture reacts at 60°C for 12 hours. Then methane iodide (2.19 g, 15.4 mmol, 961 µL, 102 eq) was also added and the reaction was continued for 1 hour. After the reaction was completed, the obtained solid mixture was added into water (30.0 mL); ethyl acetate (20 mL^{∗}3) extraction was performed; the saturated salt solution (10 mL) was used for washing; anhydrous sodium sulfate was used for drying to obtain a compound 25g.

MS-ESI calculation values [M+H]⁺ 279, 281; actual measurement values 279, 281.

### Step VI

The compound 25g (374 mg, 1.20 mmol, 5 eq) and a compound 3e (40 mg, 144 µmol, 0.6 eq) were added into anhydrous dioxane (2.5 mL) and water (0.5 mL); sodium carbonate (50.7 mg, 478 µmol, 2 eq) and 1,1-bis(diphenyl phosphorus) ferrocene palladium chloride (14.0 mg, 19.1 µmol, 0.08 eq) were further added for reaction for 1 hour at 90°C; after the reaction was completed, most of dioxane was removed through decompression and concentration; after water (20.0 mL) was added for dilution, ethyl acetate extraction (50.0 mL^{∗}3) was performed; the combined organic matter was washed with the saturated salt solution (20.0 mL), dried with anhydrous sodium sulfate, and filtered; and decompression and concentration were conducted. The residue was subjected to thin layer chromatography (1:1 petroleum ether: ethyl acetate) purification (chromatographic column: YMC CHIRAL, Amylose-C (250mm^{∗}30mm,5µm) mobile phase: [45% ethanol carbon dioxide solution (containing 0.1% ammonium hydroxide)]; flow rate: 4 mL/min; column temperature: 35°C; elution time: 4 min), and a compound 25 (retention time: 0.729 min) and a compound 26 (retention time: 2.168 min) were obtained.

### Compound 25 (retention time: 0.729 min)

MS-ESI calculation values [M+H]⁺ 511, 513; actual measurement values 511, 513.

¹H NMR (400 MHz, DMSO-*d*₆) δ:7.69 (d, *J*=9.2 Hz, 2H), 7.61 (d, *J*=9.2 Hz, 1H), 7.20 (br d, *J*=8.0 Hz, 1H), 7.06 (br s, 1H), 6.91 (br s, 1H), 6.85 (s, 1H), 6.58 (d, *J*=5.2 Hz, 1H), 6.36-6.32 (m, 1H), 6.19 (d, *J*=5.2 Hz, 1H), 4.00 (s, 3H), 3.77 (br s, 4H), 3.65 (s, 3H), 3.36-3.33 (m, 4H).

### Compound 26 (retention time: 2.168 min)

MS-ESI calculation values [M+H]⁺ 511, 513; actual measurement values 511, 513.

¹H NMR (400 MHz, DMSO-*d*6) δ:7.69 (d, *J*=9.2 Hz, 2H), 7.61 (d, *J*=9.2 Hz, 1H), 7.20 (br d, *J*=8.0 Hz, 1H), 7.07 (br s, 1H), 6.91 (br s, 1H), 6.85 (s, 1H), 6.59 (d, *J*=5.2 Hz, 1H), 6.36-6.34 (m, 1H), 6.19 (d, *J*=5.2 Hz, 1H), 4.00 (s, 3H), 3.77 (br s, 4H), 3.65 (s, 3H), 3.34 (br s, 4H).

### Experiment example 1: Evaluation in vitro DNA-PK, PI3K (p110α/p85α), PI3K (p110β/p85α), PI3K (p110σ/p85α), PI3K (p120γ) kinase inhibitory activity

This experiment was tested at Eurofins Pharma Discovery Service, Reaction Biology Corp. (RBC)

Experiment materials and method:
anthropogenic DNA-PK; Mg/ATP; GST-cMyc-p53; EDTA; Ser15 antibody; ATP: 10 µM; biotinylated phosphatidylinositol-3,4,5-triphosphate; GST label GRP1 PH domain; streptavidin and phycocyanin; europium-labelled monoclonal antibody against GST.

### Experiment method (Eurofins Pharma Discovery Service):

DNA-PK (h) was incubated in a determination buffer containing 50 nM GST-cMyc-p53 and Mg/ATP (density as required). The reaction was initiated by adding a mixture of Mg/ATP. After incubating at room temperature for 30 min, a termination solution containing EDTA was added to terminate the reaction. Finally, a detection buffer (containing labeled anti-GST monoclonal antibody and europium-labeled anti-phosphate Ser15 antibody against phosphorylated p53) was added. Then, the board was read in time-resolved fluorescence mode, and a Homogeneous Time Resolved Fluorescence (HTRF) signal was measured according to the formula HTRF = 10000×(Em665nm/Em620nm).

**PI3K** (p110α/p85α) was incubated in a determination buffer containing phosphatidylinositol 4,5-diphosphate and Mg/ATP (density as required). The reaction was initiated by adding an ATP solution. After incubating at room temperature for 30 min, a termination solution containing EDTA and biotinylated phosphatidylinositol-3,4,5-triphosphate was added to terminate the reaction. Finally, a detection buffer (containing labeled anti-GST monoclonal antibody, PH domain of GRP1 with GST label and streptavidin and phycyanin and europium-labeled anti-phosphate Ser15 antibody against phosphorylated p53) was added. Then, the board was read in time-resolved fluorescence mode, and an HTRF signal was measured according to the formula HTRF = 10000×(Em665nm/Em620nm).

**PI3K** (p110β/p85α) was incubated in a determination buffer containing phosphatidylinositol 4,5-diphosphate and Mg/ATP (density as required). The reaction was initiated by adding a mixture of Mg/ATP. After incubating at room temperature for 30 min, a termination solution containing EDTA and biotinylated phosphatidylinositol-3,4, 5-triphosphate was added to terminate the reaction. Finally, a detection buffer (containing labeled anti-GST monoclonal antibody, PH domain of GRP1 with GST label and streptavidin and phycyanin and europium-labeled anti-phosphate Ser15 antibody against phosphorylated p53) was added. Then, the board was read in time-resolved fluorescence mode, and an HTRF signal was measured according to the formula HTRF = 10000×(Em665nm/Em620nm).

PI3K (p110σ/p85α) was incubated in a determination buffer containing phosphatidylinositol 4,5-diphosphate and Mg/ATP (density as required). The reaction was initiated by adding a mixture of Mg/ATP. After incubating at room temperature for 30 min, a termination solution containing EDTA and biotinylated phosphatidylinositol-3,4, 5-triphosphate was added to terminate the reaction. Finally, a detection buffer (containing labeled anti-GST monoclonal antibody, PH domain of GRP1 with GST label and streptavidin and phycyanin and europium-labeled anti-phosphate Ser15 antibody against phosphorylated p53) was added. Then, the board was read in time-resolved fluorescence mode, and an HTRF signal was measured according to the formula HTRF = 10000×(Em665nm/Em620nm).

PI3K (p120γ) was incubated in a determination buffer containing phosphatidylinositol 4,5-diphosphate and Mg/ATP (density as required). The reaction was initiated by adding a mixture of Mg/ATP. After incubating at room temperature for 30 min, a termination solution containing EDTA and biotinylated phosphatidylinositol-3,4,5-triphosphate was added to terminate the reaction. Finally, a detection buffer (containing labeled anti-GST monoclonal antibody, PH domain of GRP1 with GST label and streptavidin and phycyanin and europium-labeled anti-phosphate Serl5 antibody against phosphorylated p53) was added. Then, the board was read in time-resolved fluorescence mode, and an HTRF signal was measured according to the formula HTRF = 10000×(Em665nm/Em620nm).

### Experiment result:

**Table 1 DNA-PK kinase activity test result**

| test substance (compound obtained from each embodiment) | DNA-PK kinase inhibitory activity IC₅₀ (nM) | PI3K (p110α/p85α) Kinase inhibitory activity IC₅₀ (nM) | PI3K (p110β/p85α) Kinase inhibitory activity IC₅₀ (nM) | PI3K (p110σ/p85α) Kinase inhibitory activity IC₅₀ (nM) | PI3K (p120γ) Kinase inhibitory activity IC₅₀ (nM) |
|---|---|---|---|---|---|
| M3814 | 1 | 42 | 33 | 14 | 1223 |
| Embodiment 1/2 | 82/0.5 | -/39 | -/- | 13 | -/- |
| Embodiment 3/4 | 3.5/587 | 509/- | -/- | 132/- | -/- |
| Embodiment 5/6 | 307/8 | -/- | -/- | -/- | -/- |
| Embodiment 7/8 | 1.5/138 | -/- | -/- | -/- | -/- |
| Embodiment 9/10 | 840/0.5 | -/- | -/- | -/- | -/- |
| Embodiment 11/12 | 4.7/657 | -/- | -/- | -/- | -/- |
| Embodiment 13/14 | 141/2.7 | -/- | -/- | -/- | -/- |
| Embodiment 15/16 | 71/4250 | -/- | -/- | -/- | -/- |
| Embodiment 17/18 | 15/33 | -/- | -/- | -/- | -/- |
| Embodiment 19/20 | 2.1/136 | 483/- | 345/- | 600/- | 9151/- |
| Embodiment 21/22 | 76/0.8 | -/43 | -/- | -/47 | -/- |
| Embodiment 23/24 | 8.7/5.7 | -/- | -/- | -/- | -/- |
| Embodiment 25/26 | 13100/ND | -/- | -/- | -/- | -/- |

Conclusion: The compound of the present disclosure has significant or even unexpected DNA-PK kinase inhibitory activity and higher DNA-PK kinase selectivity.

Note:
"ND" indicates that IC₅₀ is not detected.
"-" indicates that it is not detected.

### Experiment example 2: Pharmacokinetic evaluation

### 1. Experiment method

The tested compound was mixed with 10%DMSO/50%PEG400/40% water, vortexized and ultrasonic to prepare 0.2 mg/mL or 0.4 mg/mL approximately clarified solution, which was filtered by a microporous membrane and then standby. Balb/c female mice of 18-20 g were selected and given the candidate compound solution intravenously at a dose of 1 or 2 mg/kg. The tested compound was mixed with 10%DMSO/50%PEG400/40% water, vortexized and ultrasonic to prepare 0.2mg/mL or 1 mg/mL approximately clarified solution, which was filtered by a microporous membrane and then standby. Balb/c female mice of 18 to 20 g were selected and given orally the candidate compound solution at a dose of 2 or 10 mg/kg. Whole blood was collected for a certain period of time, and plasma was prepared. Drug concentration was analyzed by LC-MS/MS method, and pharmacokinetic parameters were calculated by Phoenix WinNonlin software (US Pharsight company).

Each parameter definition:
Co: instantaneous concentration required after intravenous injection; Cₘₐₓ: the highest blood concentration after administration; Tₘₐₓ: time required to reach peak concentration after administration; T_{1/2}: the time required for blood drug concentration to decrease by half; V_{dss}: apparent volume of distribution, which refers to the ratio constant of drug dosage to blood concentration when a drug reaches dynamic equilibrium in vivo. Cl: clearance rate, referring to drug apparent distribution volume number removed from the body per unit time; Tiast: time of last detection point; AUC₀₋ₗₐₛₜ: an area under a drug duration curve, referring to an area surrounded by the blood drug concentration curve on the time axis; Bioavailability: a measure of the rate and extent of drug absorption into the blood circulation, which was an important index to evaluate drug absorption degree.

Test result:
The test result is shown in tables 2-6.

**Table 2 Pharmacokinetic (PK) parameters in plasma of compound of embodiments**

| PK parameters in plasma of reference compound M3814 (Nedisertib) | | | | |
|---|---|---|---|---|
| PK parameters | IV(1 mg/kg) | IV(2 mg/kg) | PO(2 mg/kg) | PO(10 mg/kg) |
| C₀ (nM) | 1966 | 2156 | -- | -- |
| Cₘₐₓ (nM) | -- | -- | 580 | 2060 |
| Tₘₐₓ (h) | -- | -- | 0.25 | 0.500 |
| T_{1/2} (h) | 0.707 | 0.478 | 2.14 | 1.43 |
| V_{dss} (L/kg) | 1.34 | 1.90 | -- | -- |
| Cl (mL/min/kg) | 25.9 | 49.0 | -- | -- |
| Tₗₐₛₜ (h) | 6.00 | 4.00 | 8.00 | 8.00 |
| AUC₀₋ₗₐₛₜ (nM.h) | 1334 | 1408 | 688 | 3729 |
| Bioavailability (%) | -- | -- | 25.8 | 54 |

**Table 3 PK parameters in plasma of compound of embodiments**

| Embodiment 2 PK parameters in plasma of compound | | |
|---|---|---|
| PK parameters | IV(2 mg/kg) | PO(10 mg/kg) |
| C₀ (nM) | 1811 | -- |
| Cₘₐₓ (nM) | -- | 2525 |
| Tₘₐₓ (h) | -- | 1.00 |
| T_{1/2} (h) | 1.33 | 2.47 |
| V_{dss} (L/kg) | 2.25 | -- |
| Cl (mL/min/kg) | 20.3 | -- |
| Tₗₐₛₜ (h) | 8.00 | 8.00 |
| AUC₀₋ₗₐₛₜ (nM.h) | 3354 | 9073 |
| Bioavailability (%) | -- | 60.8 |

**Table 4 PK parameters in plasma of compound of embodiments**

| Embodiment 3 PK parameters in plasma of compound | | |
|---|---|---|
| PK parameters | IV(1 mg/kg) | PO(2 mg/kg) |
| C₀ (nM) | 1263 | -- |
| Cₘₐₓ (nM) | -- | 459 |
| Tₘₐₓ (h) | -- | 0.500 |
| T_{1/2} (h) | 1.27 | 2.83 |
| V_{dss} (L/kg) | 1.42 | -- |
| Cl (mL/min/kg) | 17.9 | -- |
| Tₗₐₛₜ (h) | 8.00 | 8.00 |
| AUC₀₋ₗₐₛₜ (nM.h) | 1855 | 1787 |
| Bioavailability (%) | -- | 48.2 |

**Table 5 Pharmacokinetic (PK) parameters in plasma of compound of embodiments**

| Embodiment 19 PK parameters in plasma | | | | |
|---|---|---|---|---|
| PK parameters | IV(1 mg/kg) | IV(2 mg/kg) PO(2 mg/kg) | | PO(10 mg/kg) |
| C₀ (nM) | 1286 | 2250 | -- | -- |
| Cₘₐₓ (nM) | -- | -- | 448 | 2745 |
| Tₘₐₓ (h) | -- | -- | 0.25 | 1.00 |
| T_{1/2} (h) | 1.31 | 1.72 | 8.14 | 1.78 |
| V_{dss} (L/kg) | 2.13 | 2.00 | -- | -- |
| Cl (mL/min/kg) | 20.2 | 16.3 | -- | -- |
| Tₗₐₛₜ (h) | 8.00 | 8.00 | 8.00 | 12.0 |
| AUC₀₋ₗₐₛₜ (nM.h) | 1569 | 3832 | 1503 | 13737 |
| Bioavailability (%) | -- | -- | 47.9 | 70.5 |

**Table 6 PK parameters in plasma of compound of embodiments**

| Embodiment 22 PK parameters in plasma of compound | | |
|---|---|---|
| PK parameters | IV(1 mg/kg) | PO(2 mg/kg) |
| C₀ (nM) | 1380 | -- |
| Cₘₐₓ (nM) | -- | 456 |
| Tₘₐₓ (h) | -- | 1.00 |
| T_{1/2} (h) | 1.89 | 6.25 |
| V_{dss} (L/kg) | 2.32 | -- |
| Cl (mL/min/kg) | 15.4 | -- |
| Tₗₐₛₜ (h) | 8.00 | 8.00 |
| AUC₀₋ₗₐₛₜ (nM.h) | 1982 | 1983 |
| Bioavailability (%) | -- | 50.0 |

| | | |
|---|---|---|
| "--" refers to not tested or no data is obtained. | | |

Experiment conclusion: the tested compound exhibits longer half-life, lower clearance rate and higher drug exposure, and has better pharmacokinetic properties in vivo than the reference compounds.

Experiment example 3: in vivo pharmacodynamics of human small cell lung cancer NCI-H69 cell subcutaneous xenograft tumor BALB/c nude mouse model:

Experiment purpose: Study of the tested compound of in vivo pharmacodynamics of human small cell lung cancer NCI-H69 cell subcutaneous xenograft tumor BALB/c nude mouse model:

Experimental animals: Female BALB/c nude mice, 6-8 weeks old, weighing 15-22 grams; supplier: Shanghai Linchang Biotechnology Co., LTD. (Linchang, Shanghai)

Experiment method and steps:

### 3.1 Cell culture

Human small cell lung cancer NCI-H69 cells (ATCC, item number: HTB-119 and ECACC-95111733) were cultured in vitro in a culture condition of RPMI-1640 medium with 10% fetal bovine serum, 100 U/mL penicillin and 100 µg/mL streptomycin, incubated at 37°C and 5% CO₂. Conventional passage was carried out twice a week. When cell saturation was 80%-90% and the number reaches the requirement, cells were collected, counted and inoculated.

### 3.2 Tumor cell inoculation (tumor inoculation)

0.2 mL (10×10⁶) NCI-H69 cells (with matrix glue, and volume ratio of 1:1) were subcutaneously inoculated into a right back of each mouse, and group administration was initiated when the average tumor volume reaches about 110-120 mm³.

### 3.3 Preparation of the test substance:

The tested compound was prepared as a 5 mg/mL clarified solution with 10%DMSO+50% polyethylene glycol 400+40% water as the solvent.

### 3.4 Tumor measurement and experimental indicators

The experimental indicators refers to study whether tumor growth was inhibited, delayed, or cured. Tumor diameter was measured twice a week with a vernier caliper. The calculation formula of the tumor volume was V = 0.5a×b², and a and b represent the long diameter and short diameter of the tumor, respectively.

The antitumor efficacy of the compound was evaluated by TGI (%) or relative tumor proliferation rate T/C (%). TGI (%) reflects the tumor growth inhibition rate. The calculation of TGI (%): TGI (%)=[1- (average tumor volume at the end of administration of a certain treatment group - average tumor volume at the beginning of administration of the treatment group)/(average tumor volume at the end of treatment in solvent control group - average tumor volume at the beginning of treatment in solvent control group)]×control group.

Relative tumor proliferation rate T/C (%): the calculation formula was as follows: T/C % = the level at the beginning of treatment in the TRTV/CRTV group (TRTV: treatment group RTV; CRTV: negative control group RTV). According to results of tumor measurement, Relative Tumor Volume (RTV) was calculated by the formula RTV=Vₜ/V₀, where Vo was the average tumor volume measured in group administration (i.e., do), and Vt was the average tumor volume measured in a certain single measurement. TRTV and CRTV collect data on the same day.

At the end of the experiment, tumor weight will be measured and T/ weight percentage will be calculated. T weight and C weight represent tumor weight of the drug administration group and the solvent control group, respectively.

### 3.5 Statistic analysis

Statistic analysis includes an average value and a standard error (SEM) of tumor volume at each time point for each group. The treatment group shows the best treatment effect on day 21 after administration at the end of the experiment, and therefore, statistic analysis was performed to assess differences among groups based on this data. T-test was used for comparison between two groups, one-way ANOVA was used for comparison among three or more groups, and Games-Howell method was used for verification if F value shows a significant difference. If there was no significant difference in F value, Dunnet (2-sided) method was applied to analysis. All data were analyzed using SPSS 17.0. P < 0.05 was considered as having a significant difference.

### 3.6 Experiment result and discussion

(1) Compared with the solvent group, in the nude mouse xenograft model of human breast cancer, M3814 (40 mg/kg, PO, QD) + etoposide (10 mg/kg, IP, 3-day administration, 4-day withdrawal), embodiment 2 (40 mg/kg, PO, QD) + etoposide (10 mg/kg, intraperitoneal, 3-day, 4-day withdrawal), embodiment 19 (40 mg/kg, PO, QD) + etoposide (10 mg/kg, IP, 3-day, 4-day withdrawal), and embodiment 19 (80 mg/kg, PO, QD) + etoposide (10 mg/kg, IP, 3-day administration, 4-day withdrawal) were significantly different from the solvent control group, with TGI of 74%, 89%, 64%, and 80%, respectively. Weight changes of various groups were respectively: M3814 (40 mg/kg, -16.00%; embodiment 2 (40 mg/kg, -16.66%), embodiment 19 (40 mg/kg, -5.88%, and embodiment 19 (80 mg/kg, 0.13%). Embodiment 19 has significant tumor suppressive effect and higher safety.

**Table 7 Tumor suppressive effect of the tested compound on human small cell lung cancer NCI-H69 cell subcutaneous xenograft tumor**

| **Group** | **Tumor volume (mm³)^{a} (0^{th} day)** | **Tumor volume (mm³)^{a} (22^{th} day)** | **RTV (22^{th} day)** | **T/C^{b} (%)** | **TGI^{b} (%)** | ***p*** |
|---|---|---|---|---|---|---|
| Solvent group | 119±11 | 1021±13 7 | 8.60 | - | - | - |
| M3814 (40 mg/kg, PO, QD) + etoposide (10 mg/kg, IP, 3-day administration, 4-day withdrawal) | 119±13 | 352±26 | 2.96 | 34.40 | 74.21 | 0.022 |
| Embodiment 2 (40 mg/kg, PO, QD) + etoposide (10 mg/kg, IP, 3-day administration, 4-day withdrawal) | 119±13 | 219±44 | 1.84 | 21.37 | 88.96 | 0.008 |
| Embodiment 19 (40 mg/kg, PO, QD) + etoposide (10 mg/kg, IP, 3-day administration, 4-day withdrawal) | 119±12 | 444±34 | 3.73 | 43.34 | 63.98 | 0.039 |
| Embodiment 19 (40 mg/kg, PO, QD) + etoposide (10 mg/kg, IP, 3-day administration, 4-day withdrawal) | 119±12 | 299±31 | 2.51 | 29.20 | 80.09 | 0.015 |

Note:
"--" needs no calculation.
a. Average value ± SEM
b. Tumor growth inhibition was calculated by T/C and TGI (TGI (%) = [1-(T₂₁-T₀)/(V₂₁-V₀)] ×100).
c. p value was based on tumor volume.

(2) When administration for 24 days, for M3814 (40 mg/kg, PO, QD) + etoposide (10 mg/kg, IP, 3-day administration, 4-day withdrawal), embodiment 19 (40 mg/kg, PO, QD) + etoposide (10 mg/kg, IP, 3-day administration, 4-day withdrawal), and embodiment 19 (40 mg/kg, PO, BID) + etoposide (10 mg/kg, IP, 3-day administration, 4-day withdrawal), T/C was separately 43.1%, 38.6%, and 28.4%, and TGI was separately 66.1%, 70.5%, and 81.5%, all of which have significant inhibitory effect on tumor growth, and as compared with the solvent control group, all of them, p < 0.05. For M3814 (40 mg/kg, QD), etoposide (10 mg/kg), embodiment 19 (40 mg/kg, BID), embodiment 19 (10 mg/kg, BID) + etoposide (10 mg/kg, IP, 3-day administration, 4-day withdrawal), and embodiment 19 (20 mg/kg, QD) + etoposide (10 mg/kg, IP, 3-day administration, 4-day withdrawal), T/C was separately 84.1%, 75.6%, 85.8%, 61.2%, and 50.6%, and TGI was separately 19.8%, 33.4%, 16.8%, 46.5%, and 56.8%, as compared with the solvent control group, p value was separately 0.603, 0.570, 0.471, 0.005, and <0.001, and all of them have a certain inhibitory effect on tumor.

The T/C values calculated by tumor weight and volume were close and trend to be consistent. The results above suggest that, in the nude mouse xenograft model of human small cell lung cancer NCI-H69, combination of M3814 (40 mg/kg, PO, QD) + etoposide (10 mg/kg IP, 3-day administration, 4-day withdrawal) has significant antitumor effect. In addition, the combination groups of embodiment 19 (40 mg/kg, PO, QD) + etoposide (10 mg/kg, IP, 3-day administration, 4-day withdrawal) and embodiment 19 (40 mg/kg, PO, BID) + etoposide (10 mg/kg, IP, 3-day administration, 4-day withdrawal) also have the significant antitumor effect, and the antitumor effect of the latter has a dose-dependent trend (upon comparison between the high-dose group and the low-dose group, P < 0.05). P values of embodiment 19 (40 mg/kg, PO, BID) + etoposide (10 mg/kg, IP, administration at first three days in one week, withdrawal at last four days in one week) were all < 0.05 as compared with the two corresponding monotherapy groups, indicating a synergistic effect.

**Table 8 Tumor suppressive effect of the tested compound on human small cell lung cancer NCI-H69 xenograft tumor model**

| **Group** | **Tumor volume (mm³)^{a} (0^{th} day)** | **Tumor volume (mm³)^{a} (24^{th} day)** | **RTV (24^{th} day)** | **TGI (%) (24^{th} day)** | **T/C (%) (24^{th} day)** |
|---|---|---|---|---|---|
| Vehicle | 114±7 | 925±93 | 8.05±0.49 | -- | -- |
| M3814+etoposide (40 mg/kg, QD + 10 mg/kg) | 114±8 | 389±28 | 3.47±0.24 | 66.1 | 43.1 |
| M3814 (40 mg/kg, QD) | 114±8 | 764±59 | 6.77±0.45 | 19.8 | 84.1 |
| Etoposide (10 mg/kg) | 114±9 | 654±48 | 6.09±0.85 | 33.4 | 75.6 |
| Embodiment 19 (40 mg/kg, BID) | 114±7 | 789±49 | 6.91±0.18 | 16.8 | 85.8 |
| Embodiment 19 + etoposide (10 mg/kg, BID + 10 mg/kg) | 114±9 | 548±44 | 4.93±0.42 | 46.5 | 61.2 |
| Embodiment 19 + etoposide (20 mg/kg, QD + 10 mg/kg) | 114±7 | 464±41 | 4.08±0.32 | 56.8 | 50.6 |
| Embodiment 19 + etoposide (40 mg/kg, QD + 10 mg/kg) | 114±7 | 353±33 | 3.11±0.18 | 70.5 | 38.6 |
| Embodiment 19 + etoposide (40 mg/kg, BID + 10 mg/kg) | 114±10 | 264±37 | 2.28±0.26 | 81.5 | 28.4 |

Note: a. Average value ± SEM, n=9.

Conclusion: The results of two in vivo efficacy experiments show that compound in embodiment 19 combined with etoposide has an obvious synergistic effect and a significant tumor suppressive effect.

### Experiment example 4: In vivo pharmacodynamics of human head and neck cancer FaDu cell subcutaneous xenograft tumor BALB/c nude mouse model:

Experiment purpose: The antitumor activity of the tested compound was evaluated in a nude mouse model of human head and neck cancer FaDu cell subcutaneous xenograft tumor

Experimental animals: Female BALB/c nude mice, 6-8 weeks old, weighing 17-23 grams; supplier: B&K Universal Group Limited

Experiment method and steps:

### 4.1 Cell culture

Human head and neck cancer FaDu cell (ATCC, Manassas, Virginiaite, item number: HTB-43) was cultured in vitro in a culture condition of EMEM medium with 10% fetal bovine serum, 100 U/mL penicillin and 100 µg/mL streptomycin, incubated at 37°C and 5% CO₂. Conventional digestive treatment passage was carried out using pancreatic enzyme - EDTA twice a week.

### 4.2 Tumor cell inoculation (tumor inoculation)

Each mouse was subcutaneously inoculated with 0.1 mL (5×110⁶) FaDu cells at the right dorsal position, and when the average tumor volume reaches about 106 mm³, randomization was used for initiating drug administration.

### 4.3 Preparation of the test substance:

The tested compound was prepared as a 5 mg/mL clarified solution with 10%DMSO+50% polyethylene glycol 400+40% water as the solvent. Preparation was conducted every three days.

### 4.4 Tumor measurement and experimental indicators

The experimental indicators refers to study whether tumor growth was inhibited, delayed, or cured. Tumor diameter was measured twice a week with a vernier caliper. The calculation formula of the tumor volume was V = 0.5a ×b², and a and b represent the long diameter and short diameter of the tumor, respectively.

The antitumor efficacy of the compound was evaluated by TGI (%) or relative tumor proliferation rate T/C (%). TGI (%) reflects the tumor growth inhibition rate. The calculation of TGI (%): TGI (%)=[1- (average tumor volume at the end of administration of a certain treatment group - average tumor volume at the beginning of administration of the treatment group)/(average tumor volume at the end of treatment in solvent control group - average tumor volume at the beginning of treatment in solvent control group)]×control group.

Relative tumor proliferation rate T/C (%): the calculation formula was as follows: T/C % = the level at the beginning of treatment in the TRTV/CRTV group (TRTV: treatment group RTV; CRTV: negative control group RTV). According to results of tumor measurement, Relative Tumor Volume (RTV) was calculated by the formula RTV=Vₜ/V₀, where Vo was the average tumor volume measured in group administration (i.e., do), and Vt was the average tumor volume measured in a certain single measurement. TRTV and CRTV collect data on the same day.

At the end of the experiment, tumor weight will be measured and T/ weight percentage will be calculated. T weight and C weight represent tumor weight of the drug administration group and the solvent control group, respectively.

### 4.5 Statistic analysis

Statistic analysis includes an average value and a standard error (SEM) of tumor volume at each time point for each group. The treatment group shows the best treatment effect on day 21 after administration at the end of the experiment, and therefore, statistic analysis was performed to assess differences among groups based on this data. T-test was used for comparison between two groups, one-way ANOVA was used for comparison among three or more groups, and Games-Howell method was used for verification if F value shows a significant difference. If there was no significant difference in F value, Dunnet (2-sided) method was applied to analysis. All data were analyzed using SPSS 17.0. P<0.05 was considered as having a significant difference.

### 4.6 Experiment result and discussion

(1) This experiment evaluates the efficacy of the compound of embodiment 19 in human head and neck cancer FaDu cell xenograft tumor model, with the solvent group as the reference. When administration for 21 days, for radiotherapy group (2 Gy, 5-day radiotherapy, 2-day withdrawal), M3814 (50 mg/kg, PO, QD) + radiotherapy group (2 Gy, 5-day radiotherapy, 2-day withdrawal), embodiment 19 (25 mg/kg, PO, QD) + radiotherapy group (2 Gy, 5-day radiotherapy, 2-day withdrawal) and embodiment 19 (50 mg/kg, PO, QD) + radiotherapy group (2 Gy, 5-day radiotherapy, 2-day withdrawal), T/C values thereof were respectively 4.35%, 0.24%, 0.08%, and 0.08%, and TGI was separately 103.91%, 108.46%, 108.61%, and 108.64%, all of which have significant inhibitory effect on tumor growth, and as compared with the solvent control group, all of them, p<0.001. For M3814 (50 mg/kg, PO, QD) and embodiment 19 (50 mg/kg, PO, QD), T/C values thereof were respectively 99.60% and 114.35%, and TGI was separately -0.43% and -15.37%, as compared with the solvent control group, p = 1.000 and 0.990, and no obvious inhibitory effect on tumor.

The results above suggest that, in the nude mouse model of human head and neck carcinoma FaDu cell transplantation, radiotherapy group (2 Gy, 5-day radiotherapy, 2-day withdrawal) and the combination group of M3814 (50 mg/kg, PO, QD) + radiotherapy group (2 Gy, 5-day radiotherapy, 2-day withdrawal) has a significant antitumor effect. In addition, the combination groups of embodiment 19 (25 mg/kg, PO, QD) + radiotherapy group (2 Gy, 5-day radiotherapy, 2-day withdrawal) and embodiment 19 (50 mg/kg, PO, QD) + radiotherapy group (2 Gy, 5-day radiotherapy, 2-day withdrawal) both have the significant antitumor effect. See Table 9 and Table 10 for details.

(2) In this model, there was no significant decrease in vivo weight of all the animals in the administration group, while the body weights of the animals in the four irradiation groups decrease slightly, but the average value was less than 10%. Half of non-irradiated animals show ulceration and scab on day 21 after group administration. One animal in Group 3 (M3814, 50 mg/kg) was found dead on day 23 after group administration. One animal in Group 4 (embodiment 19, 50 mg/kg) meets the criteria for euthanasia due to tumor ulceration and was euthanized 22 days after the start of administration. All remaining animals in Group 1 (Vehicle), Group 3 (M3814, 50 mg/kg), and Group 4 (embodiment 19, 50 mg/kg) were euthanized 24 days after the start of administration due to an average tumor volume approaching 2000 mm3.

**Table 9: Tumor suppressive effect of cancer human head and neck FaDu cell xenograft tumor model**

| **Group** | **Tumor volume (mm³)^{a}** | **Tumor volume (mm³)^{a}** | **RTV** | **TGI (%)** | **T/C (%)** |
|---|---|---|---|---|---|
| | **(0^{th} day)** | **(21^{th} day)** | **(21th day)** | **(21th day)** | **(21th day)** |
| Solvent group | 106±5 | 1315±168 | 12.40±1.51 | -- | -- |
| Radiotherapy group, 2Gy | 106±4 | 58±28 | 0.54±0.25 | 103.91 | 4.35 |
| M3814, 50 mg/kg | 106±5 | 1320±174 | 12.35±1.10 | -0.43 | 99.60 |
| Embodiment 19, 50 mg/kg | 106±5 | 1501±242 | 14.18±2.04 | -15.37 | 114.35 |
| M3814+Radiotherapy group, 50 mg/kg+2Gy | 106±5 | 3±2 | 0.03±0.02 | 108.46 | 0.24 |
| Embodiment 19+radiotherapy group, 25 mg/kg+2Gy | 106±6 | 1±1 | 0.01±0.01 | 108.61 | 0.08 |
| Embodiment 19+radiotherapy group, 50 mg/kg + 2Gy | 106±5 | 1+1 | 0.01±0.01 | 108.64 | 0.08 |

Note: a. Average value ± SEM, n=8.

T

**able 10 Tumor volume at different time points in each group**

| **Days after administration** | **Tumor volume (mm³)^{a}** | | | | | | |
|---|---|---|---|---|---|---|---|
| | **Solvent group** | **Radiothera py group 2Gy** | **M3814 50 mg/kg** | **Embodime nt 19 50 mg/kg** | **M3814+Radiother apy group 50 mg/kg+2Gy** | **Embodiment 19+radiothera py group 25 mg/kg + 2Gy** | **Embodiment 19+radiothera py group 50 mg/kg + 2Gy** |
| 0 | 106±5 | 106±4 | 106+5 | 106±5 | 106±5 | 106±6 | 106±5 |
| 3 | 167±12 | 188±13 | 167±20 | 189±18 | 168±10 | 172+16 | 175±14 |
| 7 | 356±42 | 165±10 | 322±35 | 375±28 | 106±19 | 136±11 | 155±17 |
| 10 | 447±45 | 136+18 | 502±48 | 568±50 | 68±16 | 76±11 | 97±16 |
| 14 | 745±86 | 90±27 | 752±64 | 946±108 | 25±9 | 29±5 | 45±10 |
| 17 | 968±11 1 | 71±31 | 997±10 9 | 1120±131 | 12±4 | 11±3 | 12±5 |
| 21 | 1315±1 68 | 58±28 | 1320±1 74 | 1501±242 | 3±2 | 1±1 | 1±1 |
| 23 | 1703±2 41 | 33±16 | 1831±2 54 | 1808±372 | 1±1 | 1±1 | 1±1 |
| 28 | - | 25±16 | - | - | 1±1 | 0±0 | 1±0 |
| 31 | - | 24±19 | - | - | 0±0 | 0±0 | 0±0 |
| 35 | - | 39±37 | - | - | 0±0 | 0±0 | 0±0 |
| 42 | - | 49±46 | - | - | 0±0 | 0±0 | 0±0 |
| 49 | - | 94±87 | - | - | 0±0 | 0±0 | 0±0 |
| 56 | - | 177±156 | - | - | 0±0 | 0±0 | 0±0 |
| 63 | - | 296±273 | - | - | 0±0 | 0±0 | 0±0 |
| 70 | - | 222±190 | - | - | 0±0 | 0±0 | 0±0 |

Note: a. Average value ± SEM, n=8.

Discussion: In this experiment, separate irradiation groups and combination groups both have significantly inhibitory effect on tumor growth; during the period of treatment, an average tumor volume of the combination groups was always less than that of the separate irradiation groups, and all the average tumor volumes of the combination groups were reduced to zero 31 days after grouping; and on the same day, the average tumor volume of the separate irradiation group reaches the minimum value 24 mm³. Within 7 weeks after drug withdrawal, tumor rebound was observed in the separately irradiated animals, while the tumors in the combination groups disappear completely without rebound, which suggests that a relatively low dose of DNA-PK inhibitors can enhance the therapeutic effect of radiotherapy, and the combination therapy has a long-term tumor suppressive effect.

## Claims

1. A compound of formula (I), an isomer thereof or a pharmaceutically acceptable salt thereof, 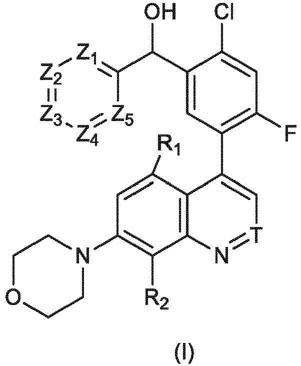 wherein,
T is CH, CR₃ or N;
Z₁, Z₂, Z₃, Z₄, and Z₅ are separately independently N or CR₄;
R₁ and R₂ are separately independently H, F, Cl, Br, I, OH or NH₂;
R₃ is F, Cl, Br, I, OH, NH₂, CN, C₁₋₆ alkyl or C₁₋₆ alkoxy, and C₁₋₆ alkyl and C₁₋₆ alkoxy are optionally substituted by 1, 2, or 3 Ra;
R₄ is independently H, F, Cl, Br, I, OH, NH₂, CN, C₁₋₆ alkyl or C₁₋₆ alkoxy, and C₁₋₆ alkyl and C₁₋₆ alkoxy are optionally substituted by 1, 2, or 3 R_{b}; and
Ra and R_{b} are separately independently F, Cl, Br, I, OH or NH₂,
wherein when T is CH and R₂ is F, then R₁ is F, Cl, Br, I, OH or NH₂.

2. The compound, the isomer thereof or the pharmaceutically acceptable salt thereof according to claim 1, wherein R₁ and R₂ are separately independently H, Cl, or F.

3. The compound, the isomer thereof or the pharmaceutically acceptable salt thereof according to claim 1, wherein R₃ is F, Cl, Br, I, OH, NH₂, CN, C₁₋₃ alkyl or C₁₋₃ alkoxy, and the C₁₋₃ alkyl and C₁₋₃ alkoxy are optionally substituted by 1, 2, or 3 Ra.

4. The compound, the isomer thereof or the pharmaceutically acceptable salt thereof according to claim 3, wherein R₃ is F, Cl, Br, I, OH, NH₂, CN, CH₃, CH₂CH₃ or OCH₃, and the CH₃, CH₂CH₃ and OCH₃ are optionally substituted by 1, 2, or 3 Ra.

5. The compound, the isomer thereof or pharmaceutically acceptable salt thereof according to claim 4, wherein R₃ is F, Cl, Br, I, OH, NH₂, CN, CH₃, CH₂F, CHF₂, CF₃, CH₂CH₃ or OCH₃.

6. The compound, the isomer thereof or the pharmaceutically acceptable salt thereof according to claim 1, wherein R₄ is independently H, F, Cl, Br, I, OH, NH₂, CN, C₁₋₃ alkyl or C₁₋₃ alkoxy, and the C₁₋₃ alkyl and C₁₋₃ alkoxy are optionally substituted by 1, 2, or 3 R_{b}.

7. The compound, the isomer thereof or the pharmaceutically acceptable salt thereof according to claim 6, wherein R₄ is H, F, Cl, Br, I, OH, NH₂, CN, CH₃, CH₂CH₃, or OCH₃.

8. The compound, the isomer thereof or the pharmaceutically acceptable salt thereof according to claim 1 or 5, wherein T is CH, N, C(NH₂), C(OCH₃) or C(CHF₂).

9. The compound, the isomer thereof or the pharmaceutically acceptable salt thereof according to claim 1 or 7, wherein Z₁, Z₂, Z₃, Z₄, and Z₅ are separately independently N, CH, C(OCH₃) or C(CH₃).

10. The compound, the isomer thereof or the pharmaceutically acceptable salt thereof according to claim 9, wherein the moiety

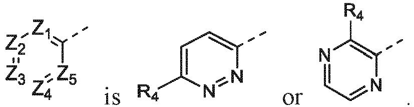

11. The compound, the isomer thereof or the pharmaceutically acceptable salt thereof according to claim 10, wherein the moiety

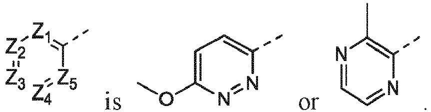

12. The compound, the isomer thereof or the pharmaceutically acceptable salt thereof according to claim 1, wherein the moiety

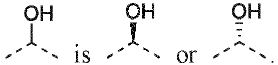

13. The compound, the isomer thereof or the pharmaceutically acceptable salt thereof according to any one of claims 1-7, which is selected from: 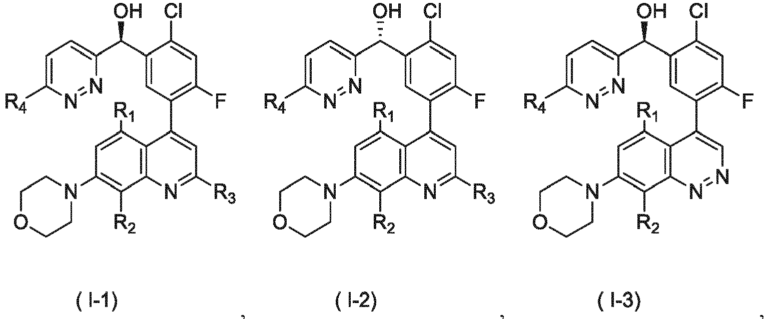 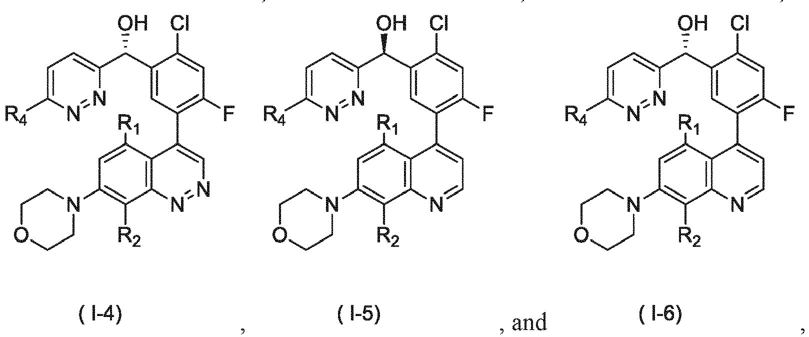 wherein,
Ri, R₂, R₃, and R₄ are defined in any one of claims 1-7.

14. A compound of the following formula, an isomer thereof or a pharmaceutically acceptable salt thereof:

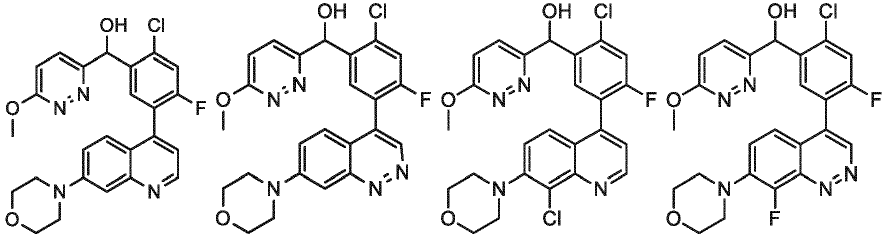

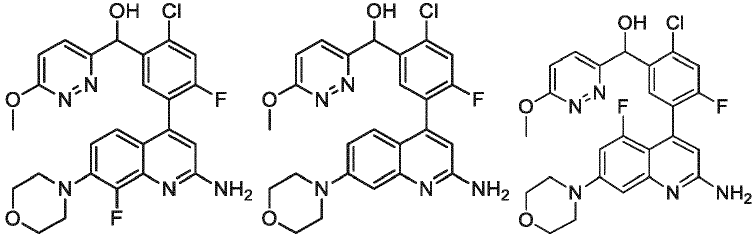

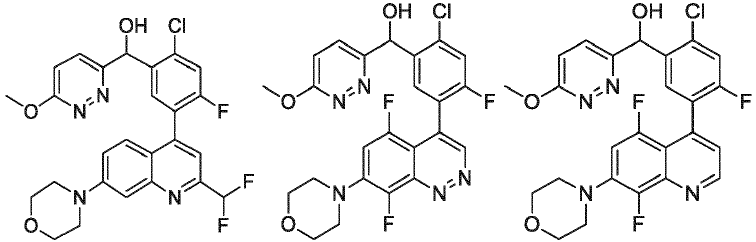

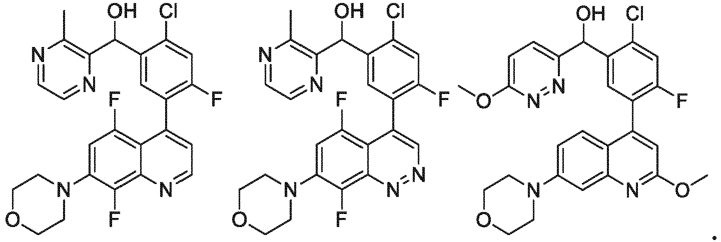

15. The compound, the isomer thereof or the pharmaceutically acceptable salt thereof according to claim 14, which is selected from:

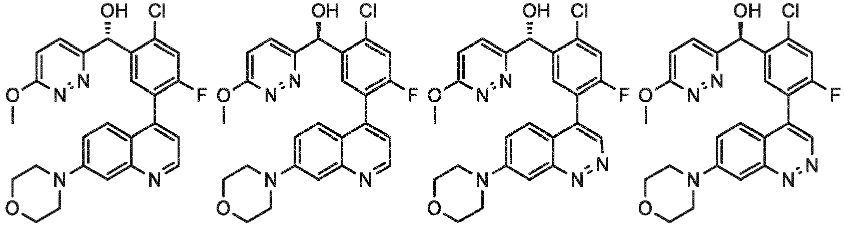

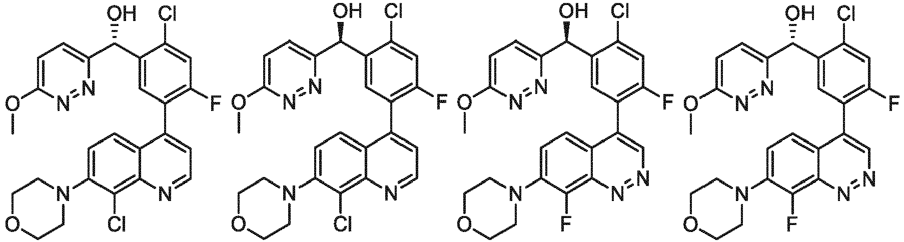

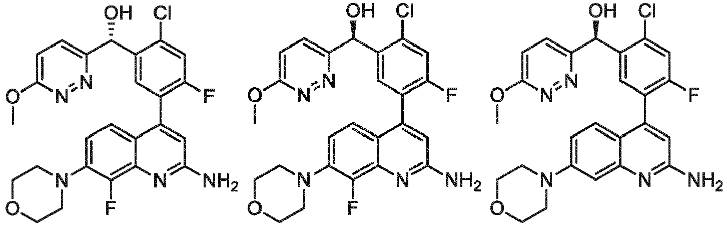

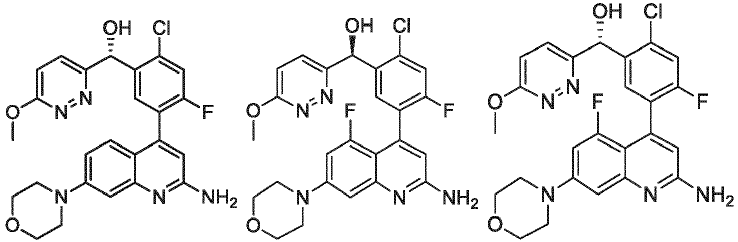

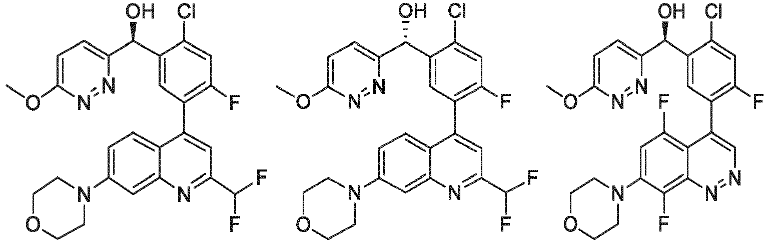

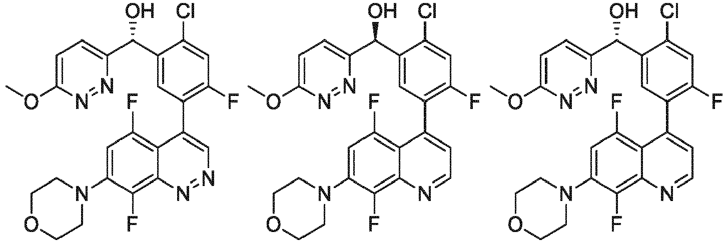

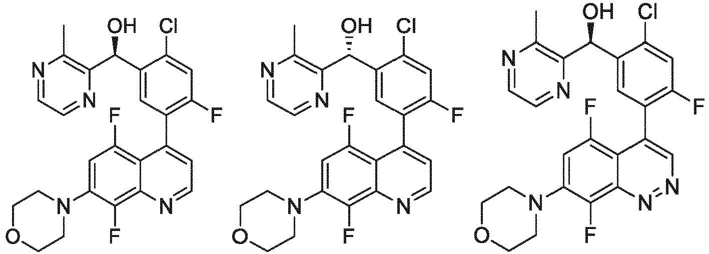

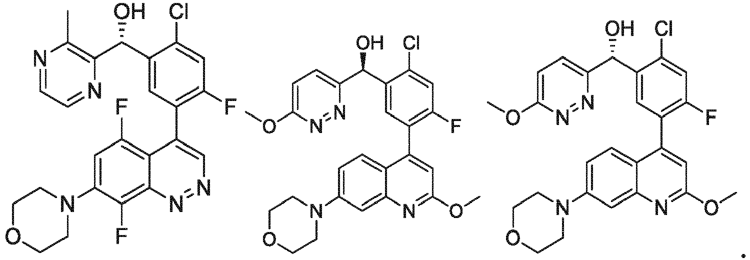

16. A pharmaceutical composition, comprising a therapeutically effective amount of the compound, the pharmaceutically acceptable salt thereof or the isomer thereof according to any one of claims 1-15 as active ingredients, and a pharmaceutically acceptable carrier.

17. Use of the compound, the isomer thereof or the pharmaceutically acceptable salt thereof according to any one of claims 1-15 or the pharmaceutical composition according to claim 16 in the manufacture a drug for treating DNA-PK related diseases.

18. The use according to claim 17, wherein the DNA-PK related drug is a drug for treating tumors.
